# FASCICULE DE BREVET EUROPEEN

(11) **EP 0 666 250 B1**
(45) Date de publication et mention de la délivrance du brevet: **11.02.1998**
(21) Numéro de dépôt: 95400216.8
(22) Date de dépôt: 02.02.1995
(51) Int. Cl.: C07C 233/78, C07C 233/88, C07D 217/04, C07D 471/04, C07D 495/04, C07D 209/44, C07D 223/16, A61K 31/165, A61K 31/47, A61K 31/435, A61K 31/38, A61K 31/40, A61K 31/55

(54) **Dérivés de N-(3-aminopropyl)-N-phényl-5,6,7,8-tétrahydronaphtalène-2-carboxamide ayant des propriétés neuroprotectrices et anti-ischémiques, leur préparation et leur application en thérapeutique**
Derivate des N-(3-Aminopropyl)-N-phenyl-5,6,7,8-tetrahydronaphthalen-2-carboxamides mit neuroprotektiven und antischaemischen Eigenschaften, ihre Herstellung und ihre therapeutische Verwendung
Derivatives of N-(3-aminopropyl)-N-phenyl-5,6,7,8-tetrahydronaphthalene-2-carboxamide having neuroprotective and anti-ischemic properties, their preparation and their use, in therapy

(30) Priorité: 03.02.1994 FR 9401198; 03.02.1994 FR 9401199; 03.02.1994 FR 9401200
(43) Date de publication de la demande: 09.08.1995
(73) Titulaire: SYNTHELABO, 92350 Le Plessis Robinson (FR)
(72) Inventeur: Frost, Jonathan, F-91320 Wissous (FR); George, Pascal, F-78730 St Arnoult en Yvelines (FR); Pasau, Patrick, F-92220 Bagneux (FR); Bartsch, Régine, F-92260 Fontenay aux Roses (FR); Rousselle, Corinne, F-92260 Fontenay aux Roses (FR); Williams, Paul Howard, F-75011 Paris (FR); Muller, Jean Claude, F-91390 Morsang sur Orge (FR)
(74) Mandataire: Ludwig, Jacques

(56) Documents cités:
- EP-A- 0 161 604
- EP-A- 0 233 762
- EP-A- 0 300 865
- EP-A- 0 352 613

## Description

La présente invention a pour objet des dérivés de *N*-(3-aminopropyl)-*N*-phényl-5,6,7,8-tétrahydronaphtalène-2-carboxamide, leur préparation et leur application en thérapeutique.

Les composés de l'invention répondent à la formule générale (I) 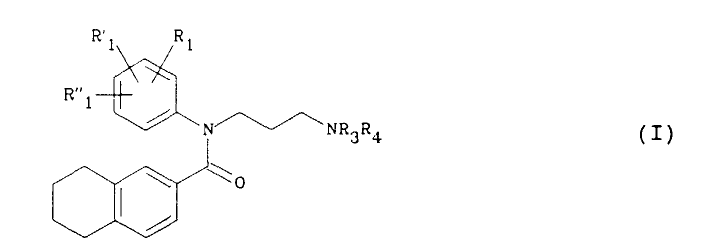 dans laquelle
R₁ représente un atome d'hydrogène ou d'halogène, un groupe méthyle ou un groupe alcoxy en C₁-C₄,
R'₁ représente un atome d'hydrogène ou d'halogène,
R"₁ représente un atome d'hydrogène ou un groupe méthoxy,
R₃, pris isolément, représente un groupe alkyle en C₁-C₃,
R₄, pris isolément, représente un groupe 2,3-dihydro-1*H*-indén-2-yle, un groupe 2,3-dihydro-1*H*-indén-1-yle ou un groupe 1,2,3,4-tétrahydronaphtalén-1-yle,
ou bien
R₃ et R₄ forment ensemble, et avec l'atome d'azote qui les porte, un groupe 1,2,3,4-tétrahydroisoquinoléin-2-yle, un groupe 6,7-diméthoxy-1,2,3,4-tétrahydroisoquinoléin-2-yle, un groupe 5,8-diméthoxy-1,2,3,4-tétrahydroisoquinoléin-2-yle, un groupe 1,2,3,4-tétrahydro-9*H*-pyrido[3,4-b]indol-2-yle, un groupe 1,2,3,4-tétrahydro-9*H*-pyrido[4,3-b]indol-3-yle, un groupe 4,5,6,7-tétrahydrothiéno[2,3-*c*]pyridin-6-yle, un groupe 2,3-dihydro-1*H*-isoindol-2-yle ou un groupe 2,3,4,5-tétrahydro-1*H*-3-benzazépin-3-yle, dont les formules respectives sont les suivantes : 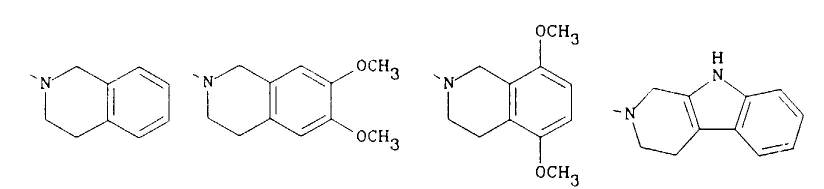 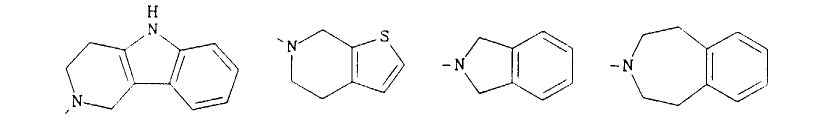 étant exclus les composés dans la formule desquels R₁ représente un groupe alcoxy, R'₁ et R"₁ représentent chacun un atome d'hydrogène, et R₃ et R₄ forment, avec l'atome d'azote, un groupe 1,2,3,4-tétrahydroisoquinoléin-2-yle substitué ou non substitué.

Parmi les composés de l'invention on préfère ceux dans la formule desquels R₁ représente un atome d'halogène, R'₁ représente un atome d'hydrogène ou d'halogène, R"₁ représente un atome d'hydrogène, R₃, pris isolément, représente un groupe alkyle en C₁-C₃, R₄, pris isolément, représente un groupe 2,3-dihydro-1*H*-indén-2-yle, un groupe 2,3-dihydro-1*H*-indén-1-yle ou un groupe 1,2,3,4-tétrahydronaphtalén-1-yle, ou bien R₃ et R₄ forment ensemble, et avec l'atome d'azote qui les porte, un groupe 1,2,3,4-tétrahydroisoquinoléin-2-yle, un groupe 6,7-diméthoxy-1,2,3,4-tétrahydroisoquinoléin-2-yle ou un groupe 5,8-diméthoxy-1,2,3,4-tétrahydroisoquinoléin-2-yle.

Parmi ces composés préférés, les plus intéressants sont le *N*-[3-[(2,3-dihydro-1*H*-indén-2-yl)méthylamino]propyl]-*N*-(3,4-dichlorophényl)-5,6,7,8-tétrahydronaphtalène-2-carboxamide, le *N*-[3-[(2,3-dihydro-1*H*-indén-1-yl)méthylamino]propyl]-*N*-(3,4-dichlorophényl)-5,6,7,8-tétrahydronaphtalène-2-carboxamide et le *N*-[3-[(1,2,3,4-tétrahydronaphtalén-1-yl)méthylamino]propyl]-*N*-(3,4-dichlorophényl)-5,6,7,8-tétrahydronaphtalène-2-carboxamide.

Les composés de l'invention peuvent exister à l'état de bases ou de sels d'addition à des acides.

Par ailleurs, lorsque R₄ contient une atome de carbone asymétrique, c'est-à-dire lorsque R₄ représente un groupe 2,3-dihydro-1*H*-indén-1-yle ou un groupe 1,2,3,4-tétrahydronaphtalén-1-yle, les composés peuvent exister sous forme d'isomères optiques purs ou de mélanges de tels isomères.

Les composés de formule générale (I) peuvent être préparés selon un procédé illustré par le schéma qui suit.

On fait réagir une benzènamine de formule générale (II), dans laquelle R₁, R'₁ et R''₁ sont tels que définis ci-dessus, avec le 5,6,7,8-tétrahydronaphtalène-2-carboxylate de méthyle, de formule (III), par exemple en présence d'hydrure de sodium et dans un solvant tel que le diméthylsulfoxyde.
On obtient un amide de formule générale (IV), qu'on fait réagir ensuite avec le 1-bromo-3-chloropropane, de formule (V), par exemple en présence d'hydrure de sodium et dans un solvant tel que le *N*,*N*-diméthylformamide.
On obtient un dérivé chloré, de formule générale (VI), qu'on fait finalement réagir avec une amine de formule générale HNR₃R₄, dans laquelle R₃ et R₄ sont tels que définis ci-dessus, par exemple en présence d'iodure de potassium et d'une base telle que le carbonate de potassium, dans un solvant tel que le *N*,*N*-diméthylformamide.

Les benzènamines de formule générale (II) sont disponibles dans le commerce, décrites dans la littérature, par exemple dans les demandes de brevets EP-0144730 et EP-0300865, ou accessibles par des méthodes décrites dans la littérature ou connues de l'homme du métier.

Le 5,6,7,8-tétrahydronaphtalène-2-carboxylate de méthyle est décrit dans *J. Amer. Chem. Soc.* (1943) **65** 1097.

Les amines de formule générale (VII) dans laquelle R₄ représente un groupe 2,3-dihydro-1*H*-indén-2-yle sont décrites dans *J. Med. Chem.* (1980) **23** 745.

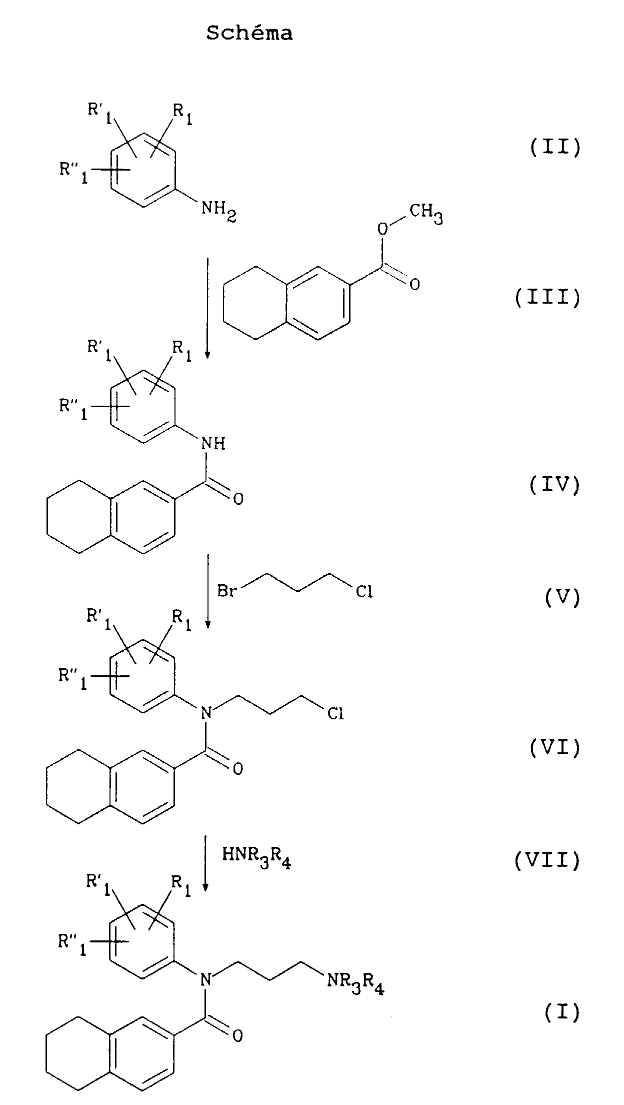

Les amines de formule générale (VII) dans laquelle R₄ représente un groupe 2,3-dihydro-1*H*-indén-1-yle sont décrites dans *J. Amer. Chem. Soc.* (1966) **88** 2233.

Les amines de formule générale (VII) dans laquelle R₄ représente un groupe 1,2,3,4-tétrahydronaphtalén-1-yle sont décrites dans *J. Amer. Chem. Soc.* (1960) **82** 459, dans *C. R. Hebd. Séances Acad. Sci. Ser. C.* (1969) **268** 2225 et dans *J. Med. Chem.* (1966) **9** 830.

Les 1,2,3,4-tétrahydroisoquinoléin-2-yles correspondant à la formule générale (VII) sont disponibles dans le commerce ou décrites dans la littérature.

Le 1,2,3,4-tétrahydro-9*H*-pyrido[3,4-*b*]indole correspondant à la formule générale (VII) est décrit dans *Organic Synthesis* (1971) **51** 136.

Le 1,2,3,4-tétrahydro-9*H*-pyrido[4,3-*b*]indole correspondant à la formule générale (VII) est décrit dans *J. Chem. Soc.* (1968)1235.

La 4,5,6,7-tétrahydrothiéno[2,3-*c*]pyridine correspondant à la formule générale (VII) est décrite dans *Arkiv. Kemi* (1970) **13**(19) 217.

Le 2,3-dihydro-1*H*-isoindole correspondant à la formule générale (VII) est décrit dans *Organic Synthesis Coll.* (1973) **5** 406.

La 2,3,4,5-tétrahydro-1*H*-3-benzazépine correspondant à la formule générale (VII) est décrite dans *Helv. Chim. Acta* (1935) **18** 1388.

Les exemples qui vont suivre illustrent en détail la préparation de quelques composés selon l'invention.
Les microanalyses élémentaires, et les spectres I.R. et R.M.N. confirment les structures des composés obtenus.
Les numéros indiqués entre parenthèses dans les titres des exemples correspondent à ceux de la 1ère colonne du tableau donné plus loin.

### Exemple 1 (Composé N°2)

Ethanedioate (1:1) et (*E*)-2-butènedioate (1:1) de *N*-[3-[(2,3-dihydro-1*H*-indén-2-yl)méthylamino]propyl]-*N*-(3,4-dichlorophényl)-5,6,7,8-tétrahydronaphtalène-2-carboxamide.

### 1.1. N-(3,4-Dichlorophényl)-5,6,7,8-tétrahydronaphtalène-2-carboxamide.

A une suspension de 1,008 g (0,021 mole) d'hydrure de sodium (à 50% dans l'huile) dans 8 ml de diméthylsulfoxyde, sous atmosphère d'argon, on ajoute une goutte de méthanol, on laisse agiter le mélange pendant 10 min, et on ajoute 1,94 g (0,012 mole) de 3,4-dichlorobenzènamine. On agite le mélange pendant 15 min, on ajoute, goutte à goutte, 2,0 g (0,0105 mole) de 5,6,7,8-tétrahydronaphtalène-2-carboxylate de méthyle en solution dans 8 ml de diméthylsulfoxyde, et on maintient l'agitation à température ambiante pendant 3h.
On ajoute lentement 150 ml d'eau, 50 ml d'éther diéthylique et 50 ml d'acétate d'éthyle, on sépare la phase organique, on la lave successivement avec 50 ml d'eau et 50 ml de solution aqueuse saturée de chlorure de sodium, on la sèche sur sulfate de magnésium, on la filtre et on évapore le solvant.
On fait cristalliser le résidu dans un mélange d'éther diéthylique et d'hexane, et on obtient 2,09 g de produit qu'on utilise tel quel dans l'étape suivante.

### 1.2. N-(3-Chloropropyl)-N-(3,4-dichlorophényl)-5,6,7,8-tétrahydronaphtalène-2-carboxamide.

A une solution de 2,05 g (0,0064 mole) de *N*-(3,4-dichlorophényl)-5,6,7,8-tétrahydronaphtalène-2-carboxamide dans 11 ml de *N,N*-diméthylformamide, sous atmosphère d'azote, on ajoute lentement, par petites portions, 0,369 g (0,0077 mole) d'une suspension d'hydrure de sodium à 50% dans l'huile, on refroidit le mélange à 0°C, on ajoute, goutte à goutte, 1,26 g (0,008 mole) de 1-bromo-3-chloropropane, on laisse revenir à température ambiante et on maintient l'agitation pendant 4h.
On refroidit le mélange, on ajoute lentement 50 ml d'eau et 50 ml d'éther diéthylique, on sépare les phases, et on extrait la phase aqueuse avec 50 ml d'éther diéthylique. On réunit les phases organiques pour les laver successivement avec deux fois 50 ml d'eau, une fois 50 ml d'acide chlorhydrique 1N, deux fois 50 ml d'eau et une fois 50 ml de solution aqueuse saturée de chlorure de sodium, on sèche sur sulfate de magnésium, et on évapore le solvant.
On obtient 2,51 g de produit qu'on utilise tel quel dans l'étape suivante.

### 1.3. Ethanedioate (1:1) de N-[3-[(2,3-dihydro-1H-indén-2-yl)méthylamino]propyl]-N-(3,4-dichlorophényl)-5,6,7,8-tétrahydronaphtalène-2-carboxamide.

A une solution de 2,45 g (0,0062 mole) de *N*-(3-chloropropyl)-*N*-(3,4-dichlorophényl)-5,6,7,8-tétrahydronaphtalène-2-carboxamide dans 9 ml de *N*,*N*-diméthylformamide, sous atmosphère d'argon, on ajoute 1,71 g (0,0124 mole) de carbonate de potassium et 1,03 g (0,0062 mole) d'iodure de potassium puis, après 5 min, 1,14 g (0,0062 mole) de chlorhydrate de *N*-méthyl-2,3-dihydro-1*H*-indène-2-amine, et on chauffe le mélange à 85°C pendant 4h.
On le laisse refroidir, on ajoute 50 ml d'eau et 50 ml d'éther diéthylique, on sépare les phases, et on extrait la phase aqueuse avec deux fois 50 ml d'éther diéthylique. On réunit les phases organiques pour les laver avec 50 ml de solution aqueuse saturée de chlorure de sodium, on sèche sur sulfate de magnésium, et on évapore le solvant.
On obtient 3,04 g de produit huileux qu'on purifie par chromatographie sur colonne de gel de silice en éluant avec un mélange 97/3 de dichlorométhane/méthanol.
On obtient 0,930 g de base pure sous forme d'huile.
On prépare l'oxalate dans le propan-2-ol en ajoutant 0,165 g (0,0018 mole) d'acide oxalique à 0,930 g (0,0018 mole) de base, on l'isole et on le recristallise dans l'acétate d'éthyle.
On obtient finalement 0,64 g d'oxalate sous forme de cristaux blancs.
Point de fusion : 140-141°C.

### 1.4. (E)-2-butènedioate (1:1) de N-[3-[(2,3-dihydro-1H-indén-2-yl)méthylamino]propyl]-N-(3,4-dichlorophényl)-5,6,7,8-tétrahydronaphtalène-2-carboxamide.

On prépare le fumarate dans un mélange de propan-2-ol et d'éther diisopropylique, en ajoutant 1,05 g (0,009 mole) d'acide fumarique à 4,58 g (0,009 mole) de base ; on isole le sel et on le recristallise dans un mélange de propan-2-ol et d'éther diisopropylique.
On obtient finalement 4,28 g de fumarate sous forme de cristaux blancs.
Point de fusion : 160-161°C.

### Exemple 2 (Composé N°13)

Ethanedioate de *N*-(4-chlorophényl)-*N*-[3-(6,7-diméthoxy-1,2,3,4-tétrahydroisoquinoléin-2-yl)propyl]-5,6,7,8-tétrahydronaphtalène-2-carboxamide.

### 2.1. N-(4-Chlorophényl)-5,6,7,8-tétrahydronaphtalène-2-car-boxamide.

A une suspension de 0,912 g (0,019 mole) d'hydrure de sodium (à 50% dans l'huile) dans 7 ml de diméthylsulfoxyde, sous atmosphère d'argon, on ajoute une goutte de méthanol, on laisse agiter le mélange pendant 10 min, et on ajoute 1,40 g (0,011 mole) de 4-chlorobenzènamine. On agite le mélange pendant 15 min, on ajoute, goutte à goutte, 1,8 g (0,0095 mole) de 5,6,7,8-tétrahydronaphtalène-2-carboxylate de méthyle en solution dans 7 ml de diméthylsulfoxyde, et on maintient l'agitation à température ambiante pendant 3h.
On ajoute lentement 150 ml d'eau, 50 ml d'éther diéthylique et 50 ml d'acétate d'éthyle, on sépare la phase organique, on la lave successivement avec 50 ml d'eau et 50 ml de solution aqueuse saturée de chlorure de sodium, on la sèche sur sulfate de magnésium, on la filtre et on évapore le solvant.
On fait cristalliser le résidu dans l'éther diéthylique, et on obtient 1,96 g de produit qu'on utilise tel quel dans l'étape suivante.

### 2.2. N-(4-Chlorophényl)-N-(3-chloropropyl)-5,6,7,8-tétrahydronaphtalène-2-carboxamide.

A une solution de 1,92 g (0,0067 mole) de *N*-(4-chlorophényl)-5,6,7,8-tétrahydronaphtalène-2-carboxamide dans 11 ml de *N,N*-diméthylformamide, sous atmosphère d'azote, on ajoute lentement, par petites portions, 0,384 g (0,0080 mole) d'une suspension d'hydrure de sodium à 50% dans l'huile, on refroidit le mélange à 0°C, on ajoute, goutte à goutte, 1,325 g (0,0084 mole) de 1-bromo-3-chloropropane, on laisse revenir à température ambiante et on maintient l'agitation pendant 4h.
On refroidit le mélange, on ajoute lentement 50 ml d'eau et 50 ml d'éther diéthylique, on sépare les phases, et on extrait la phase aqueuse avec 50 ml d'éther diéthylique. On réunit les phases organiques pour les laver successivement avec deux fois 50 ml d'eau, une fois 50 ml d'acide chlorhydrique 1N, deux fois 50 ml d'eau et une fois 50 ml de solution aqueuse saturée de chlorure de sodium, on sèche sur sulfate de magnésium, et on évapore le solvant.
On obtient 2,29 g de produit qu'on utilise tel quel dans l'étape suivante.

### 2.3. Ethanedioate de N-(4-chlorophényl)-N-[3-(6,7-diméthoxy-1,2,3,4-tétrahydroisoquinoléin-2-yl)propyl]-5,6,7,8-tétrahydronaphtalène-2-carboxamide.

A une solution de 2,29 g (0,0063 mole) de *N*-(4-chlorophényl)-*N*-(3-chloropropyl)-5,6,7,8-tétrahydronaphtalène-2-carboxamide dans 9 ml de *N,N*-diméthylformamide, sous atmosphère d'argon, on ajoute 1,738 g (0,0126 mole) de carbonate de potassium et 1,04 g (0,0063 mole) d'iodure de potassium puis, après 5 min, 1,45 g (0,0063 mole) de chlorhydrate de 6,7-diméthoxy-1,2,3,4-tétrahydroisoquinoléine, et on chauffe le mélange à 85°C pendant 4h.
On le laisse refroidir, on ajoute 50 ml d'eau et 50 ml d'éther diéthylique, on sépare les phases, et on extrait la phase aqueuse avec deux fois 50 ml d'éther diéthylique. On réunit les phases organiques pour les laver avec 50 ml de solution aqueuse saturée de chlorure de sodium, on sèche sur sulfate de magnésium, et on évapore le solvant.
On obtient 3,1 g de produit huileux qu'on purifie par chromatographie sur colonne de gel de silice en éluant avec un mélange 97/3 de dichlorométhane/méthanol.
On obtient 1,33 g de base pure sous forme d'huile.
On prépare l'oxalate dans le propan-2-ol en ajoutant 0,230 g (0,0025 mole) d'acide oxalique à 1,32 g (0,0025 mole) de base, on l'isole et on le recristallise dans le propan-2-ol. On obtient finalement 1,0 g d'oxalate sous forme de cristaux blancs.
Point de fusion : 162-163°C.

### Exemple 3 (Composé N°17)

Ethanedioate de *N*-[3-(6,7-diméthoxy-1,2,3,4-tétrahydroisoquinoléin-2-yl)propyl]-*N*-(4-méthylphényl)-5,6,7,8-tétrahydronaphtalène-2-carboxamide.

### 3.1. N-(4-méthylphényl)-5,6,7,8-tétrahydronaphtalène-2-carboxamide.

A une suspension de 0,912 g (0,019 mole) d'hydrure de sodium (à 50% dans l'huile) dans 7 ml de diméthylsulfoxyde, sous atmosphère d'argon, on ajoute une goutte de méthanol, on laisse agiter le mélange pendant 10 min, et on ajoute 1,177 g (0,011 mole) de 4-méthylbenzènamine. On agite le mélange pendant 15 min, on ajoute, goutte à goutte, 1,8 g (0,0095 mole) de 5,6,7,8-tétrahydronaphtalène-2-carboxylate de méthyle en solution dans 7 ml de diméthylsulfoxyde, et on maintient l'agitation à température ambiante pendant 3h. On ajoute lentement 150 ml d'eau, 50 ml d'éther diéthylique et 50 ml d'acétate d'éthyle, on sépare la phase organique, on la lave successivement avec 50 ml d'eau et 50 ml de solution aqueuse saturée de chlorure de sodium, on la sèche sur sulfate de magnésium, on la filtre et on évapore le solvant. On fait cristalliser le résidu dans l'éther diéthylique, et on obtient 1,43 g de produit qu'on utilise tel quel dans l'étape suivante.

### 3.2. N-(3-chloropropyl)-N-(4-méthylphényl)-5,6,7,8-tétrahydronaphtalène-2-carboxamide.

A une solution de 1,4 g (0,0053 mole) de *N*-(4-méthylphényl)-5,6,7,8-tétrahydronaphtalène-2-carboxamide dans 9 ml de *N,N*-diméthylformamide, sous atmosphère d'azote, on ajoute lentement, par petites portions, 0,307 g (0,0064 mole) d'une suspension d'hydrure de sodium à 50% dans l'huile, on refroidit le mélange à 0°C, on ajoute, goutte à goutte, 1,048 g (0,0066 mole) de 1-bromo-3-chloropropane, on laisse revenir à température ambiante et on maintient l'agitation pendant 4h.
On refroidit le mélange, on ajoute lentement 50 ml d'eau et 50 ml d'éther diéthylique, on sépare les phases, et on extrait la phase aqueuse avec 50 ml d'éther diéthylique. On réunit les phases organiques pour les laver successivement avec deux fois 50 ml d'eau, une fois 50 ml d'acide chlorhydrique 1N, deux fois 50 ml d'eau et une fois 50 ml de solution aqueuse saturée de chlorure de sodium, on sèche sur sulfate de magnésium, et on évapore le solvant.
On obtient 1,55 g de produit qu'on utilise tel quel dans l'étape suivante.

### 3.3. Ethanedioate de N-[3-(6,7-diméthoxy-1,2,3,4-tétrahydroisoquinoléin-2-yl)propyl]-N-(4-méthylphényl)-5,6,7,8-tétrahydronaphtalène-2-carboxamide.

A une solution de 1,55 g (0,0045 mole) de N-(3-chloropropyl)-*N*-(4-méthylphényl)-5,6,7,8-tétrahydronaphtalène-2-carboxamide dans 7 ml de *N,N*-diméthylformamide, sous atmosphère d'argon, on ajoute 1,24 g (0,009 mole) de carbonate de potassium et 0,747 g (0,0045 mole) d'iodure de potassium puis, après 5 min, 1,03 g (0,0045 mole) de chlorhydrate de 6,7-diméthoxy-1,2,3,4-tétrahydroisoquinoléine, et on chauffe le mélange à 85°C pendant 4h.
On le laisse refroidir, on ajoute 50 ml d'eau et 50 ml d'éther diéthylique, on sépare les phases, et on extrait la phase aqueuse avec deux fois 50 ml d'éther diéthylique. On réunit les phases organiques pour les laver avec 50 ml de solution aqueuse saturée de chlorure de sodium, on sèche sur sulfate de magnésium, et on évapore le solvant.
On obtient 2,07 g de produit huileux qu'on purifie par chromatographie sur colonne de gel de silice en éluant avec un mélange 97/3 de dichlorométhane/méthanol.
On obtient 0,920 g de base pure sous forme d'huile.
On prépare l'oxalate dans le propan-2-ol en ajoutant 0,162 g (0,0018 mole) d'acide oxalique à 0,9 g (0,0018 mole) de base, on l'isole et on le recristallise dans le propan-2-ol.
On obtient finalement 0,797 g d'oxalate sous forme de cristaux blancs.
Point de fusion : 159-160°C.

### Exemple 4 (Composé N°26)

Ethanedioate de *N*-[4-(2-méthylpropoxy)phényl]-*N*-[3-(1,2,3,4-tétrahydro-9*H*-pyrido[4,3-*b*]indol-3-yl)propyl]-5,6,7,8-tétrahydronaphtalène-2-carboxamide.

### 4.1. N-[4-(2-Méthylpropoxy)phényl]acétamide.

A une solution de 23 g (0,15 mole) de *N*-(4-hydroxyphényl)-acétamide dans 124 ml de *N*,*N*-diméthylformamide on ajoute 32,6 ml (0,3 mole) de 1-bromo-2-méthylpropane et 31 g (0,225 mole) de carbonate de potassium, et on chauffe le mélange à 100°C pendant 5h.
On le refroidit, on évapore le solvant, on reprend le résidu avec 400 ml d'éther diéthylique et 200 ml de soude 1N, on sépare la phase organique, on la lave successivement avec trois fois 50 ml de soude 1N, trois fois 100 ml d'eau et une fois 50 ml de solution aqueuse saturée de chlorure de sodium, on la sèche sur sulfate de magnésium, on la filtre et on évapore le solvant.
On obtient 30,96 g de produit qu'on utilise tel quel dans l'étape suivante.

### 4.2. 4-(2-Méthylpropoxy)benzènamine.

A une solution de 30,35 g (0,146 mole) de *N*-[4-(2-méthylpropoxy)phényl]acétamide dans 157 ml d'éthanol on ajoute 41,5 ml (0,309 mole) de soude à 30%, et on chauffe le mélange au reflux pendant 5h.
On évapore le solvant, on reprend le résidu avec 400 ml d'éther diéthylique et 350 ml d'eau, on sépare la phase organique, on la lave successivement avec trois fois 100 ml d'eau et une fois 50 ml de solution aqueuse saturée de chlorure de sodium, on la sèche sur sulfate de magnésium, on la filtre et on évapore le solvant.
On obtient 23,64 g de produit que l'on purifie par chromatographie sur colonne de gel de silice en éluant avec un mélange 7/3 de cyclohexane/acétate d'éthyle. On obtient 22,43 g de produit.

### 4.3. N-[4-(2-Méthylpropoxy)phényl]-5,6,7,8-tétrahydronaphtalène-2-carboxamide.

A une suspension de 1,25 g (0,026 mole) d'hydrure de sodium (à 50% dans l'huile) dans 10 ml de diméthylsulfoxyde, sous atmosphère d'argon, on ajoute une goutte de méthanol, on laisse agiter le mélange pendant 10 min, et on ajoute 2,6 g (0,015 mole) de 4-(2-méthylpropoxy)benzènamine. On agite le mélange pendant 15 min, on ajoute, goutte à goutte, 2,5 g (0,013 mole) de 5,6,7,8-tétrahydronaphtalène-2-carboxylate de méthyle en solution dans 10 ml de diméthylsulfoxyde, et on maintient l'agitation à température ambiante pendant 3h.
On ajoute lentement 200 ml d'eau, 100 ml d'éther diéthylique et 100 ml d'acétate d'éthyle, on sépare la phase organique, on la lave successivement avec 100 ml d'eau, 100 ml d'acide chlorhydrique 1N, deux fois 50 ml d'eau et une fois 100 ml de solution aqueuse saturée de chlorure de sodium, on la sèche sur sulfate de magnésium, on la filtre et on évapore le solvant.
On fait cristalliser le résidu dans un mélange d'éther diéthylique et d'hexane, et on obtient 3,10 g de produit qu'on utilise tel quel dans l'étape suivante.

### 4.4. N-(3-Chloropropyl)-N-[4-(2-méthylpropoxy)phényl]-5,6,7,8-tétrahydronaphtalène-2-carboxamide.

A une solution de 2,5 g (0,008 mole) de *N*-[4-(2-méthylpropoxy)phényl]-5,6,7,8-tétrahydronaphtalène-2-carboxamide dans 13 ml de *N*,*N*-diméthylformamide, sous atmosphère d'azote, on ajoute lentement, par petites portions, 0,5 g (0,010 mole) d'une suspension d'hydrure de sodium à 50% dans l'huile, on refroidit le mélange à 0°C, on ajoute, goutte à goutte, 1,6 g (0,010 mole) de 1-bromo-3-chloropropane, on laisse revenir à température ambiante et on maintient l'agitation pendant 4h.
On refroidit le mélange, on ajoute lentement 100 ml d'eau et 100 ml d'éther diéthylique, on sépare les phases, et on extrait la phase aqueuse avec 100 ml d'éther diéthylique. On réunit les phases organiques pour les laver successivement avec deux fois 50 ml d'eau, une fois 50 ml d'acide chlorhydrique 1N, deux fois 50 ml d'eau et une fois 50 ml de solution aqueuse saturée de chlorure de sodium, on sèche sur sulfate de magnésium, et on évapore le solvant.
On obtient 3,13 g de produit qu'on utilise tel quel dans l'étape suivante.

### 4.5. Ethanedioate de N-[4-(2-méthylpropoxy)phényl]-N-[3-(1,2,3,4-tétrahydro-9H-pyrido[4,3-b]indol-3-yl)propyl]-5,6,7,8-tétrahydronaphtalène-2-carboxamide.

A une solution de 3,13 g (0,008 mole) de *N*-(3-chloropropyl)-*N*-[4-(2-méthylpropoxy)phényl]-5,6,7,8-tétrahydronaphtalène-2-carboxamide dans 11 ml de *N*,*N*-diméthylformamide, sous atmosphère d'argon, on ajoute 2,21 g (0,016 mole) de carbonate de potassium et 1,33 g (0,008 mole) d'iodure de potassium puis, après 5 min, 1,67 g (0,008 mole) de chlorhydrate de 1,2,3,4-tétrahydro-9*H*-pyrido[4,3-*b*]indole, et on chauffe le mélange à 85°C pendant 4h.
On le laisse refroidir, on ajoute 100 ml d'eau et 100 ml d'éther diéthylique, on sépare les phases, et on extrait la phase aqueuse avec deux fois 50 ml d'éther diéthylique. On réunit les phases organiques pour les laver avec 100 ml de solution aqueuse saturée de chlorure de sodium, on sèche sur sulfate de magnésium, et on évapore le solvant.
On obtient 4,29 g de produit huileux qu'on purifie par chromatographie sur colonne de gel de silice en éluant avec un mélange 95/5 de dichlorométhane/méthanol.
On obtient 1,63 g de base pure sous forme d'huile.
On prépare l'oxalate dans le propan-2-ol en ajoutant 0,221 g (0,0025 mole) d'acide oxalique à 1,32 g (0,0025 mole) de base, on l'isole et on le recristallise dans le propan-2-ol.
On isole finalement 1,0 g d'oxalate sous forme de cristaux blancs.
Point de fusion : 121-122°C.

### Exemple 5 (Composé N°20)

Ethanedioate de *N*-(4-méthoxyphényl)-*N*-[3-(1,2,3,4-tétrahydro-9*H*-pyrido[3,4-*b*]indol-2-yl)propyl]-5,6,7,8-tétrahydronaphtalène-2-carboxamide.

### 5.1. N-(4-Méthoxyphényl)-5,6,7,8-tétrahydronaphtalène-2-carboxamide.

A une suspension de 1,25 g (0,026 mole) d'hydrure de sodium (à 50% dans l'huile) dans 10 ml de diméthylsulfoxyde, sous atmosphère d'argon, on ajoute une goutte de méthanol, on laisse sous agitation pendant 10 min, on ajoute 1,92 g (0,015 mole) de 4-méthoxybenzènamine, on laisse sous agitation pendant 15 min, on ajoute, goutte à goutte, 2,5 g (0,013 mole) de 5,6,7,8-tétrahydronaphtalène-2-carboxylate de méthyle en solution dans 10 ml de diméthylsulfoxyde, et on maintient l'agitation à température ambiante pendant 3h.
On ajoute lentement 200 ml d'eau, 100 ml d'éther diéthylique et 100 ml d'acétate d'éthyle, on sépare la phase organique, on la lave successivement avec 100 ml d'eau, 100 ml d'acide chlorhydrique 1N, deux fois 50 ml d'eau et une fois 100 ml de solution aqueuse saturée de chlorure de sodium, on la sèche sur sulfate de magnésium, on la filtre et on évapore le solvant.
On obtient 3,34 g de résidu qu'on fait cristalliser dans un mélange d'éther diéthylique et d'hexane.
On obtient 2,69 g de produit qu'on utilise tel quel dans l'étape suivante.

### 5.2. N-(3-Chloropropyl)-N-(4-méthoxyphényl)-5,6,7,8-tétrahydronaphtalène-2-carboxamide.

A une solution de 2,47 g (0,0088 mole) de *N*-(4-méthoxyphényl)-5,6,7,8-tétrahydronaphtalène-2-carboxamide dans 14 ml de *N,N*-diméthylformamide, sous atmosphère d'azote, on ajoute lentement, par petites portions, 0,506 g (0,0105 mole) d'une suspension d'hydrure de sodium à 50% dans l'huile, on refroidit le mélange à 0°C, on ajoute, goutte à goutte, 1,73 g (0,011 mole) de 1-bromo-3-chloropropane, on laisse revenir à température ambiante et on maintient l'agitation pendant 4h.
On refroidit le mélange, on ajoute lentement 100 ml d'eau et 100 ml d'éther diéthylique, on sépare les phases, et on extrait la phase aqueuse avec 100 ml d'éther diéthylique. On réunit les phases organiques pour les laver successivement avec deux fois 50 ml d'eau, une fois 50 ml d'acide chlorhydrique 1N, deux fois 50 ml d'eau et une fois 50 ml de solution aqueuse saturée de chlorure de sodium, on sèche sur sulfate de magnésium, et on évapore le solvant.
On obtient 3,11 g de produit qu'on utilise tel quel dans l'étape suivante.

### 5.3. Ethanedioate de N-(4-méthoxyphényl)-N-[3-(1,2,3,4-tétrahydro-9H-pyrido[3,4-b]indol-2-yl)propyl]-5,6,7,8-tétrahydronaphtalène-2-carboxamide.

A une solution de 2,28 g (0,0064 mole) de *N*-(3-chloropropyl)-*N*-(4-méthoxyphényl)-5,6,7,8-tétrahydronaphtalène-2-carboxamide dans 8 ml de *N*,*N*-diméthylformamide, sous atmosphère d'argon, on ajoute 1,76 g (0,0128 mole) de carbonate de potassium et 1,06 g (0,0064 mole) d'iodure de potassium puis, après 5 min, 1,1 g (0,0064 mole) de chlorhydrate de 1,2,3,4-tétrahydro-9*H*-pyrido[3,4-*b*]indole, et on chauffe le mélange à 85°C pendant 4h.
On le laisse refroidir, on ajoute 100 ml d'eau et 100 ml d'éther diéthylique, on sépare les phases, et on extrait la phase aqueuse avec deux fois 50 ml d'éther diéthylique. On réunit les phases organiques pour les laver avec 100 ml de solution aqueuse saturée de chlorure de sodium, on sèche sur sulfate de magnésium, et on évapore le solvant.
On obtient 2,66 g de produit huileux qu'on purifie par chromatographie sur colonne de gel de silice en éluant avec un mélange 97/3 de dichlorométhane/méthanol.
On obtient 0,930 g de base pure sous forme d'huile.
On prépare l'oxalate dans le propan-2-ol en ajoutant 0,170 g (0,0019 mole) d'acide oxalique à 0,930 g (0,0019 mole) de base, on l'isole et on le recristallise dans le propan-2-ol.
On obtient finalement 0,515 g d'oxalate sous forme de cristaux blancs.
Point de fusion : 245°C.

### Exemple 6 (Composé N°28)

Ethanedioate de *N*-(4-chlorophényl)-*N*-[3-(2,3,4,5-tétrahydro-1*H*-3-benzazépin-3-yl)propyl]-5,6,7,8-tétrahydronaphtalène-2-carboxamide.

### 6.1. N-(4-Chlorophényl)-5,6,7,8-tétrahydronaphtalène-2-carboxamide.

A une suspension de 0,912 g (0,019 mole) d'hydrure de sodium (à 50% dans l'huile) dans 7 ml de diméthylsulfoxyde, sous atmosphère d'argon, on ajoute une goutte de méthanol, on laisse sous agitation pendant 10 min, on ajoute 1,40 g (0,011 mole) de 4-chlorobenzènamine, on laisse sous agitation pendant 15 min, on ajoute, goutte à goutte, 1,8 g (0,0095 mole) de 5,6,7,8-tétrahydronaphtalène-2-carboxylate de méthyle en solution dans 7 ml de diméthylsulfoxyde, et on maintient l'agitation à température ambiante pendant 3h.
On ajoute lentement 150 ml d'eau, 50 ml d'éther diéthylique et 50 ml d'acétate d'éthyle, on sépare la phase organique, on la lave successivement avec 50 ml d'eau et 50 ml de solution aqueuse saturée de chlorure de sodium, on la sèche sur sulfate de magnésium, on la filtre et on évapore le solvant.
On obtient 2,5 g de résidu qu'on fait cristalliser dans l'éther diéthylique.
On obtient 1,96 g de produit qu'on utilise tel quel dans l'étape suivante.

### 6.2. N-(4-Chlorophényl)-N-(3-chloropropyl)-5,6,7,8-tétrahydronaphtalène-2-carboxamide.

A une solution de 1,92 g (0,0067 mole) de *N*-(4-chlorophényl)-5,6,7,8-tétrahydronaphtalène-2-carboxamide dans 11 ml de *N,N*-diméthylformamide, sous atmosphère d'azote, on ajoute lentement, par petites portions, 0,384 g (0,008 mole) d'une suspension d'hydrure de sodium à 50% dans l'huile, on refroidit le mélange à 0°C, on ajoute, goutte à goutte, 1,325 g (0,0084 mole) de 1-bromo-3-chloropropane, on laisse revenir à température ambiante et on agite pendant 4h.
On refroidit le mélange, on ajoute lentement 50 ml d'eau et 50 ml d'éther diéthylique, on sépare les phases, et on extrait la phase aqueuse avec 50 ml d'éther diéthylique. On réunit les phases organiques pour les laver successivement avec deux fois 50 ml d'eau, une fois 50 ml d'acide chlorhydrique 1N, deux fois 50 ml d'eau et une fois 50 ml de solution aqueuse saturée de chlorure de sodium, on sèche sur sulfate de magnésium, et on évapore le solvant.
On obtient 2,29 g de produit qu'on utilise tel quel dans l'étape suivante.

### 6.3. Ethanedioate de N-(4-chlorophényl)-N-[3-(2,3,4,5-tétrahydro-1H-3-benzazépin-3-yl)propyl]-5,6,7,8-tétrahydronaphtalène-2-carboxamide.

A une solution de 2,5 g (0,0069 mole) de *N*-(4-chlorophényl)-*N*-(3-chloropropyl)-5,6,7,8-tétrahydronaphtalène-2-carboxamide dans 9 ml de *N*,*N*-diméthylformamide, sous atmosphère d'argon, on ajoute 1,9 g (0,0138 mole) de carbonate de potassium et 1,14 g (0,0069 mole) d'iodure de potassium puis, après 5 min, 1,26 g (0,0069 mole) de chlorhydrate de 2,3,4,5-tétrahydro-1*H*-3-benzazépine et on chauffe le mélange à 85°C pendant 4h.
On le laisse refroidir, on ajoute 50 ml d'eau et 50 ml d'éther diéthylique, on sépare les phases, et on extrait la phase aqueuse avec deux fois 50 ml d'éther diéthylique. On réunit les phases organiques pour les laver avec 50 ml de solution aqueuse saturée de chlorure de sodium, on sèche sur sulfate de magnésium, et on évapore le solvant.
On obtient 3,04 g de produit huileux qu'on purifie par chromatographie sur colonne de gel de silice en éluant avec un mélange 97/3 de dichlorométhane/méthanol.
On obtient 1,18 g de base pure sous forme d'huile.
On-prépare l'oxalate dans le propan-2-ol en ajoutant 0,225 g (0,0025 mole) d'acide oxalique à 1,18 g (0,0025 mole) de base, on l'isole et on le recristallise dans le propan-2-ol.
On obtient finalement 1,04 g d'oxalate sous forme de cristaux blancs.
Point de fusion : 180°C.

### Exemple 7 (Composé N°27)

Ethanedioate (1:1) de *N*-(4-chlorophényl)-*N*-[3-(4,5,6,7-tétrahydrothiéno[2,3-*c*]pyridin-6-yl)propyl]-5,6,7,8-tétrahydronaphtalène-2-carboxamide.

A une solution de 2,37 g (0,0065 mole) de *N*-(4-chlorophényl)-*N*-(3-chloropropyl)-5,6,7,8-tétrahydronaphtalène-2-carboxamide dans 10 ml de *N,N*-diméthylformamide, sous atmosphère d'argon, on ajoute 2,69 g (0,0195 mole) de carbonate de potassium et 1,1 g (0,0065 mole) d'iodure de potassium puis, après 5 min, 1,49 g (0,0065 mole) d'oxalate de 4,5,6,7-tétrahydrothiéno[2,3-*c*]pyridine et on chauffe le mélange à 85°C pendant 4h.
On le laisse refroidir, on ajoute 50 ml d'eau et 50 ml d'éther diéthylique, on sépare les phases, et on extrait la phase aqueuse avec deux fois 50 ml d'éther diéthylique. On réunit les phases organiques pour les laver avec 50 ml de solution aqueuse saturée de chlorure de sodium, on sèche sur sulfate de magnésium, et on évapore le solvant.
On obtient 3,0 g de produit huileux qu'on purifie par chromatographie sur colonne de gel de silice en éluant avec un mélange 97/3 de dichlorométhane/méthanol.
On obtient 1,16 g de base pure sous forme d'huile.
On prépare l'oxalate dans le propan-2-ol en ajoutant 0,26 g (0,0022 mole) d'acide oxalique à 1,16 g (0,0022 mole) de base, on l'isole et on le recristallise dans le propan-2-ol.
On obtient finalement 1,09 g d'oxalate sous forme de cristaux blancs.
Point de fusion : 164-165°C.

### Exemple 8 (Composé N°31)

(E)-2-butènedioate (1:1) de *N*-(4-chlorophényl)-*N*-[3-(2,3-dihydro-1*H*-isoindol-2-yl)propyl]-5,6,7,8-tétrahydronaphtalène-2-carboxamide.

A une solution de 2,0 g (0,0055 mole) de *N*-(4-chlorophényl)-*N*-(3-chloropropyl)-5,6,7,8-tétrahydronaphtalène-2-carboxamide dans 10 ml de *N,N*-diméthylformamide, sous atmosphère d'argon, on ajoute 1,15 g (0,0083 mole) de carbonate de
potassium et 0,92 g (0,0055 mole) d'iodure de potassium puis, après 5 min, 0,66 g (0,0055 mole) de 2,3-dihydro-1*H*-isoindole et on chauffe le mélange à 85°C pendant 4h.
On le laisse refroidir, on ajoute 50 ml d'eau et 50 ml d'éther diéthylique, on sépare les phases, et on extrait la phase aqueuse avec deux fois 50 ml d'éther diéthylique. On réunit les phases organiques pour les laver avec 50 ml de solution aqueuse saturée de chlorure de sodium, on sèche sur sulfate de magnésium, et on évapore le solvant.
On obtient 2,8 g de produit huileux qu'on purifie par chromatographie sur colonne de gel de silice en éluant avec un mélange 97/3 de dichlorométhane/méthanol.
On obtient 0,82 g de base pure sous forme d'huile.
On prépare le fumarate dans le propan-2-ol en ajoutant 0,21 g (0,0018 mole) d'acide fumarique à 0,82 g (0,0018 mole) de base, on l'isole et on le recristallise dans le propan-2-ol.
On obtient finalement 0,43 g de fumarate sous forme de cristaux blancs.
Point de fusion : 141-142°C.

### Exemple 9 (Composé N°44)

Ethanedioate (1:1) de(±)-*N*-[3-[(2,3-dihydro-1*H*-indén-1-yl)méthylamino]propyl]-*N*-(3,4-dichlorophényl)-5,6,7,8-tétrahydronaphtalène-2-carboxamide.

A une solution de 3 g (0,0075 mole) de *N*-(3-chloropropyl)-*N*-(3,4-dichlorophényl)-5,6,7,8-tétrahydronaphtalène-2-carboxamide dans 12 ml de *N*,*N*-diméthylformamide, sous atmosphère d'argon, on ajoute 2,07 g (0,015 mole) de carbonate de potassium et 1,25 g (0,0075 mole) d'iodure de potassium puis, après 5 min d'agitation, 1,37 g (0,0075 mole) de chlorhydrate de *N*-méthyl-2,3-dihydro-1*H*-indène-1-amine, et on chauffe le mélange à 85°C pendant 3h30.
On le laisse refroidir, on ajoute 50 ml d'eau, on sépare le précipité marron par filtration, en le lavant à l'eau, et on le sèche.
On obtient 3,58 g de produit qu'on purifie par chromatographie sur colonne de gel de silice en éluant avec un mélange 96/4 de dichlorométhane/méthanol.
On obtient 1,12 g de base pure.
Pour préparer l'oxalate on dissout 0,73 g (0,0014 mole) de base et 0,13 g (0,0014 mole) d'acide oxalique dans 10 ml de propan-2-ol, on chauffe le mélange au reflux jusqu'à dissolution, on laisse refroidir, on recueille le précipité blanc et on le recristallise dans le propan-2-ol. Après filtration et séchage on obtient finalement 0,58 g d'oxalate.
Point de fusion : 142-143°C.

### Exemple 10 (Composé N°45)

Ethanedioate (1:1) de(±)-*N*-[3-[(1,2,3,4-tétrahydronaphtalén-1-yl)méthylamino]propyl]-*N*-(3,4-dichlorophényl)-5,6,7,8-tétrahydronaphtalène-2-carboxamide.

A une solution de 3 g (0,0075 mole) de *N*-(3-chloropropyl)-*N*-(3,4-dichlorophényl)-5,6,7,8-tétrahydronaphtalène-2-carboxamide dans 12 ml de *N*,*N*-diméthylformamide, sous atmosphère d'argon, on ajoute 2,07 g (0,015 mole) de carbonate de potassium et 1,25 g (0,0075 mole) d'iodure de potassium puis, après 5 min d'agitation, 1,48 g (0,0075 mole) de chlorhydrate de *N*-méthyl-1,2,3,4-tétrahydronaphtalène-1-amine, et on chauffe le mélange à 85°C pendant 3h30.
On le laisse refroidir, on ajoute 50 ml d'eau, on sépare le précipité jaune par filtration, en le lavant à l'eau, et on le sèche.
On obtient 4,32 g de produit qu'on purifie par chromatographie sur colonne de gel de silice en éluant avec un mélange 96/4 de dichlorométhane/méthanol.
On obtient 1,34 g de base pure sous forme d'huile.
Pour préparer l'oxalate on dissout les 1,34 g (0,0026 mole) de base et 0,23 g (0,0026 mole) d'acide oxalique dans 15 ml de propan-2-ol, on chauffe le mélange au reflux jusqu'à dissolution, on évapore le solvant, on recristallise le résidu dans un grand volume d'éther diisopropylique, on recueille le précipité blanc par filtration et on le sèche.
On obtient finalement 0,71 g d'oxalate.
Point de fusion : 116-117°C.

Le tableau qui suit illustre les structures chimiques et les propriétés physiques de quelques composés selon l'invention. Dans la colonne "Sel", "-" désigne un composé à l'état de base, "ox." désigne un oxalate, ou éthanedioate, "fum." désigne un fumarate, ou (*E*)-2-butènedioate, "cit." désigne un citrate, ou 2-hydroxy-1,2,3-propanetricarboxylate, et "més." désigne un mésotartrate, ou 2,3-dihydroxybutanedioate ; le rapport indiqué entre parenthèses est le rapport molaire acide:base.

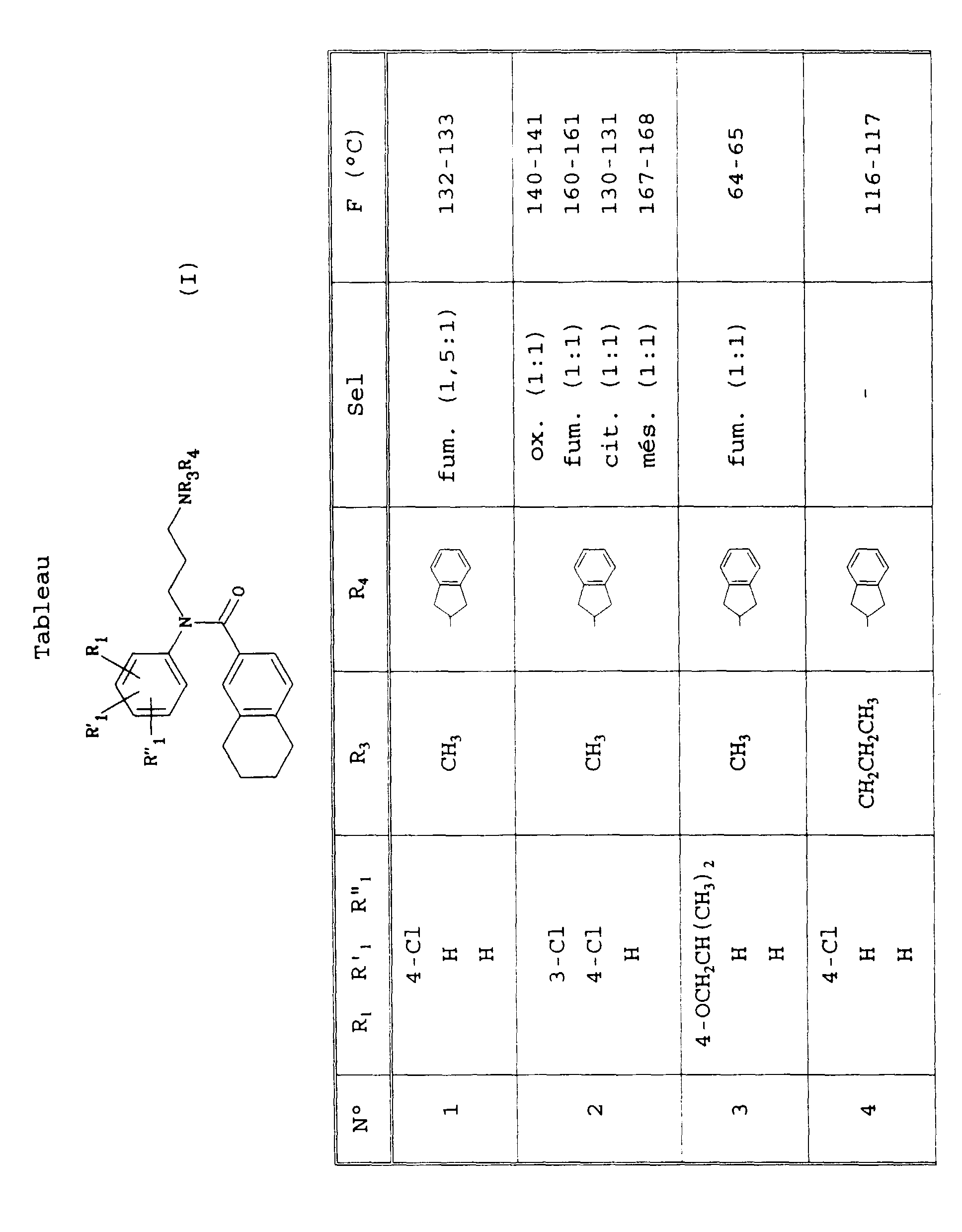

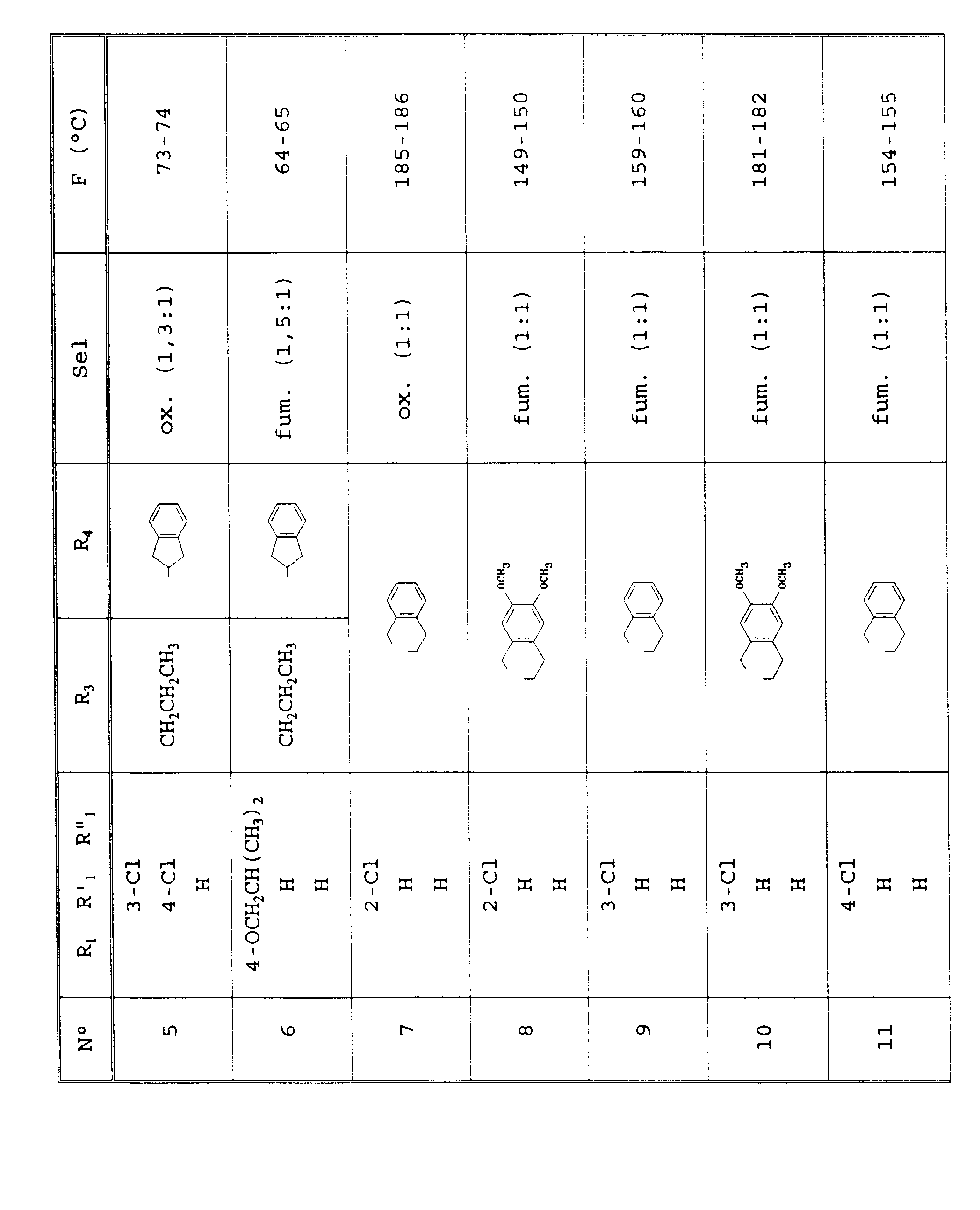

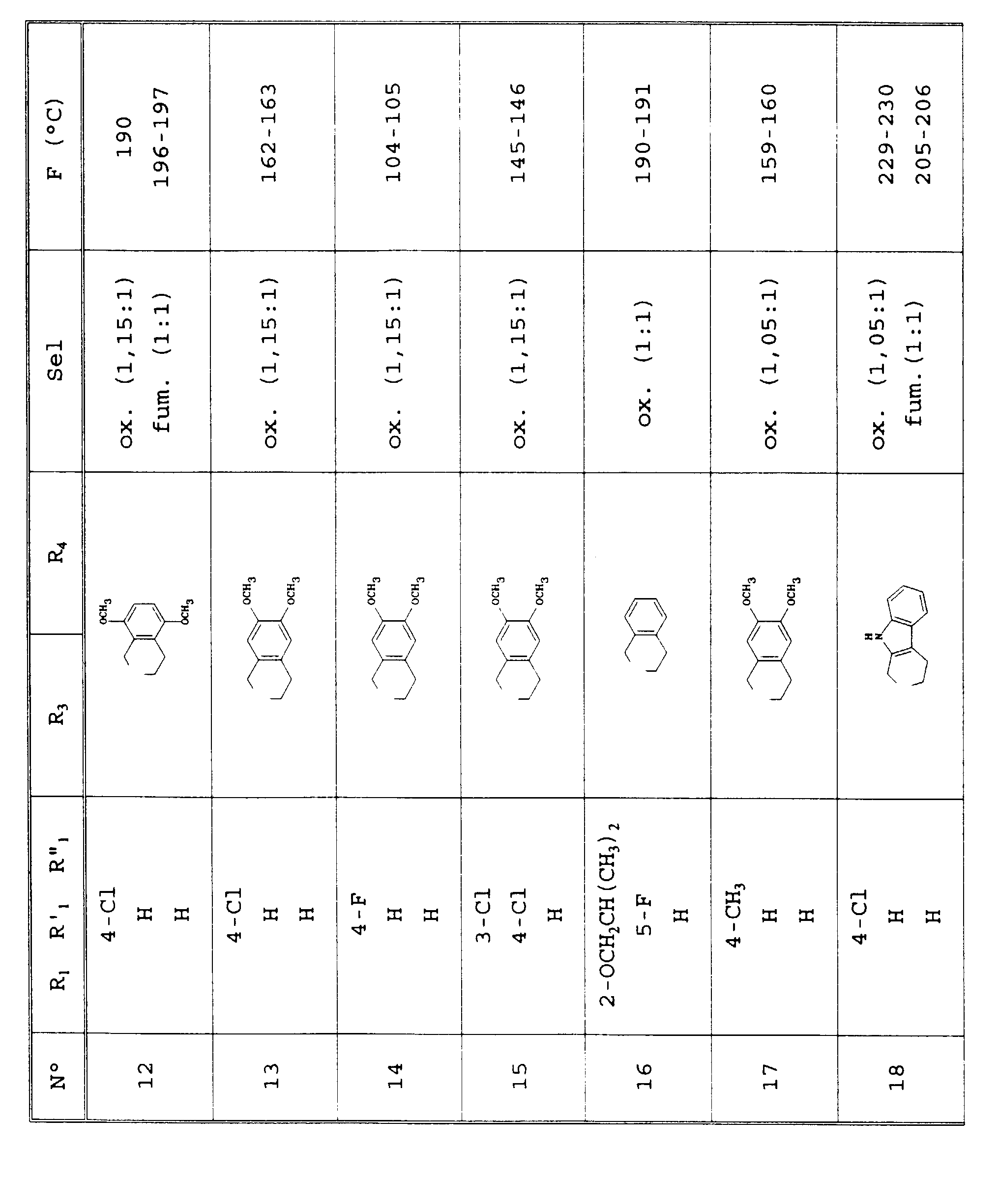

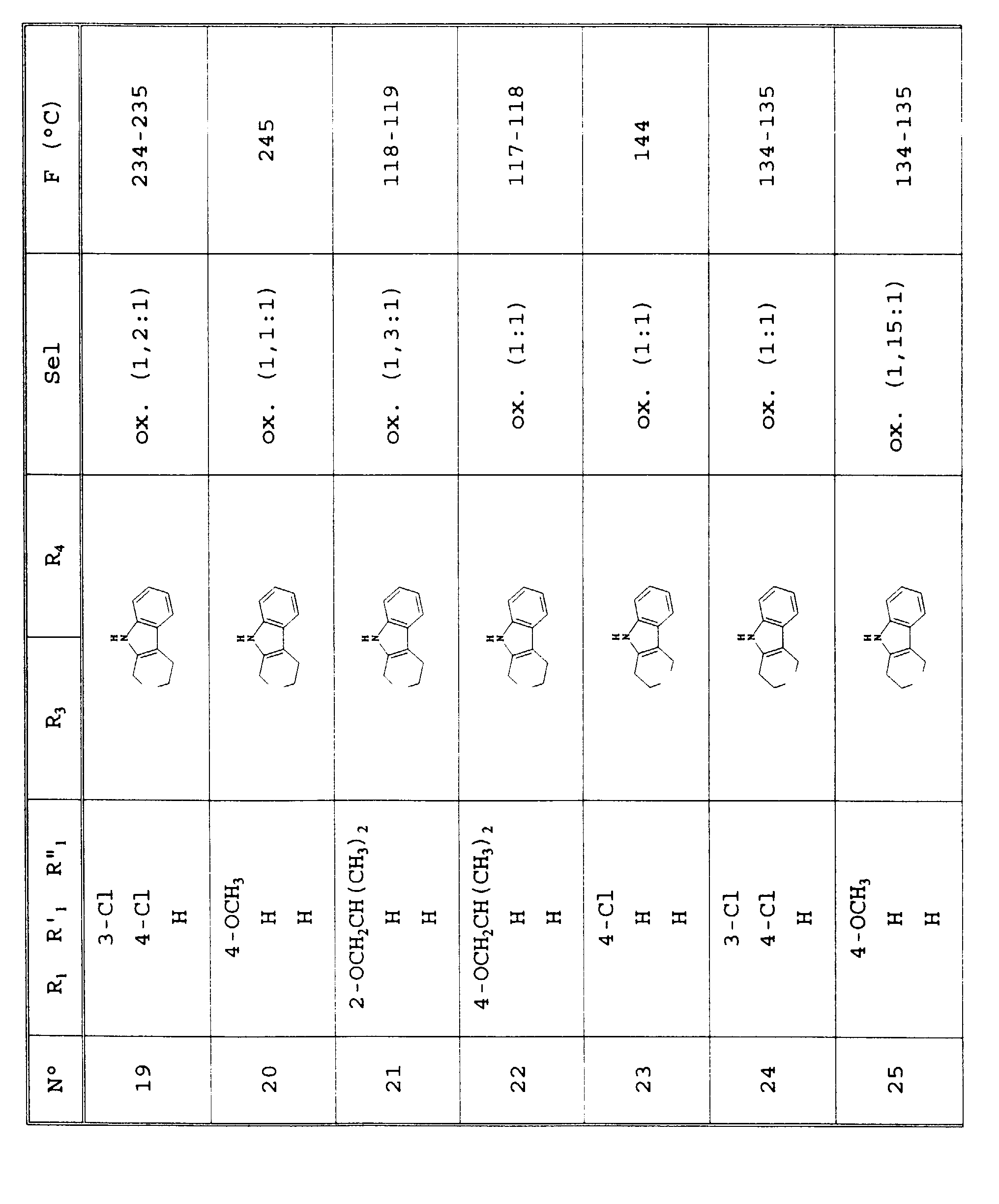

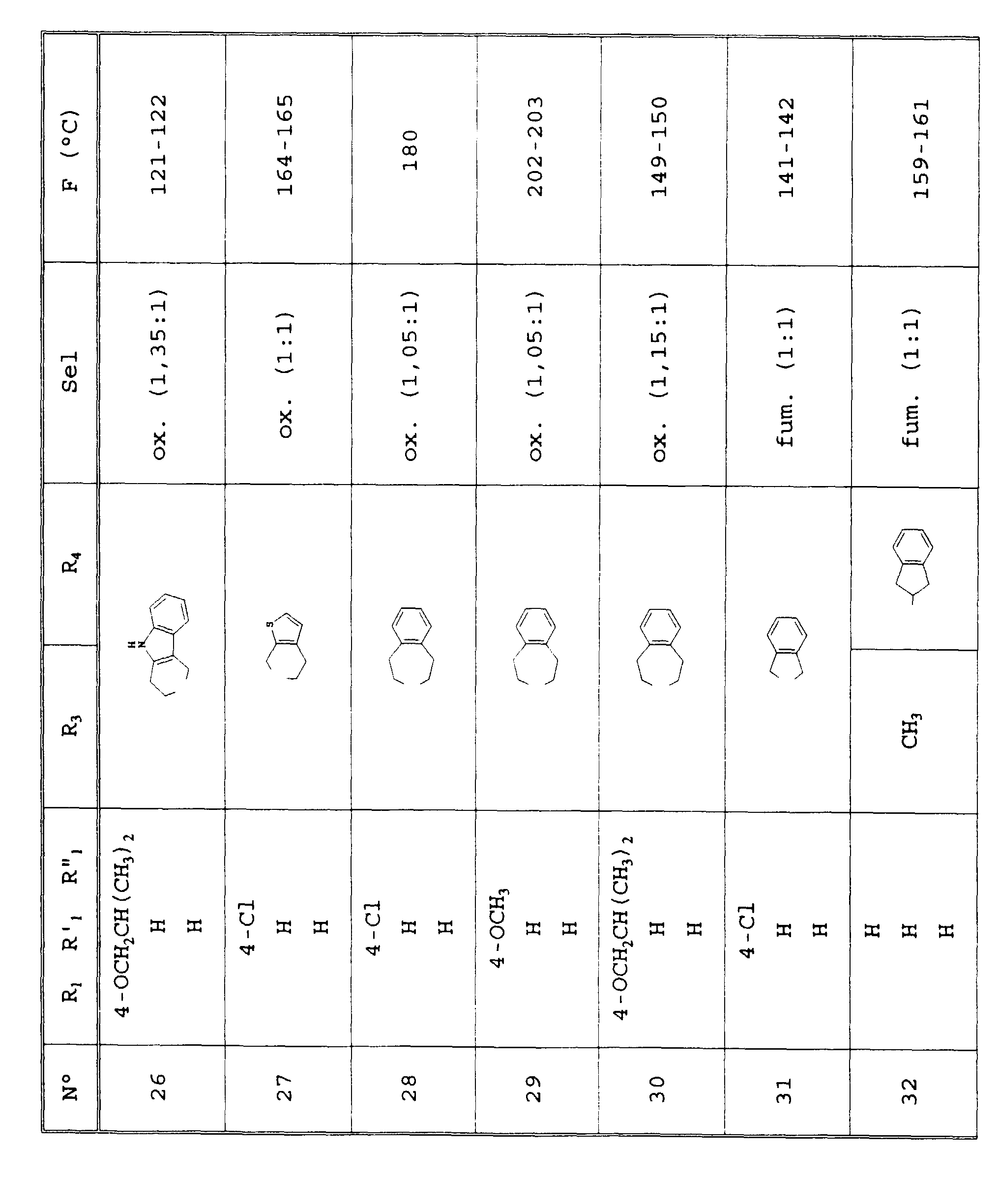

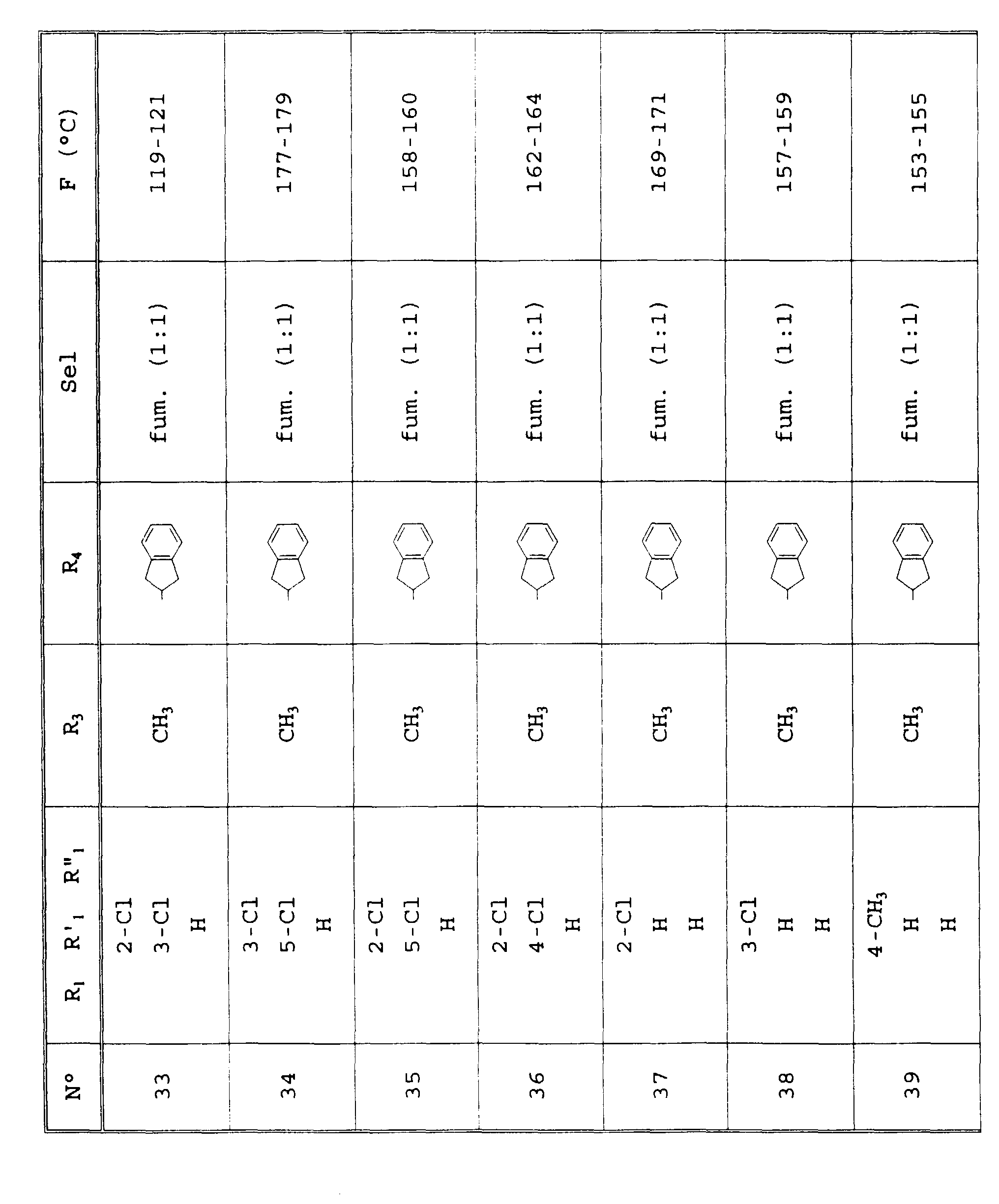

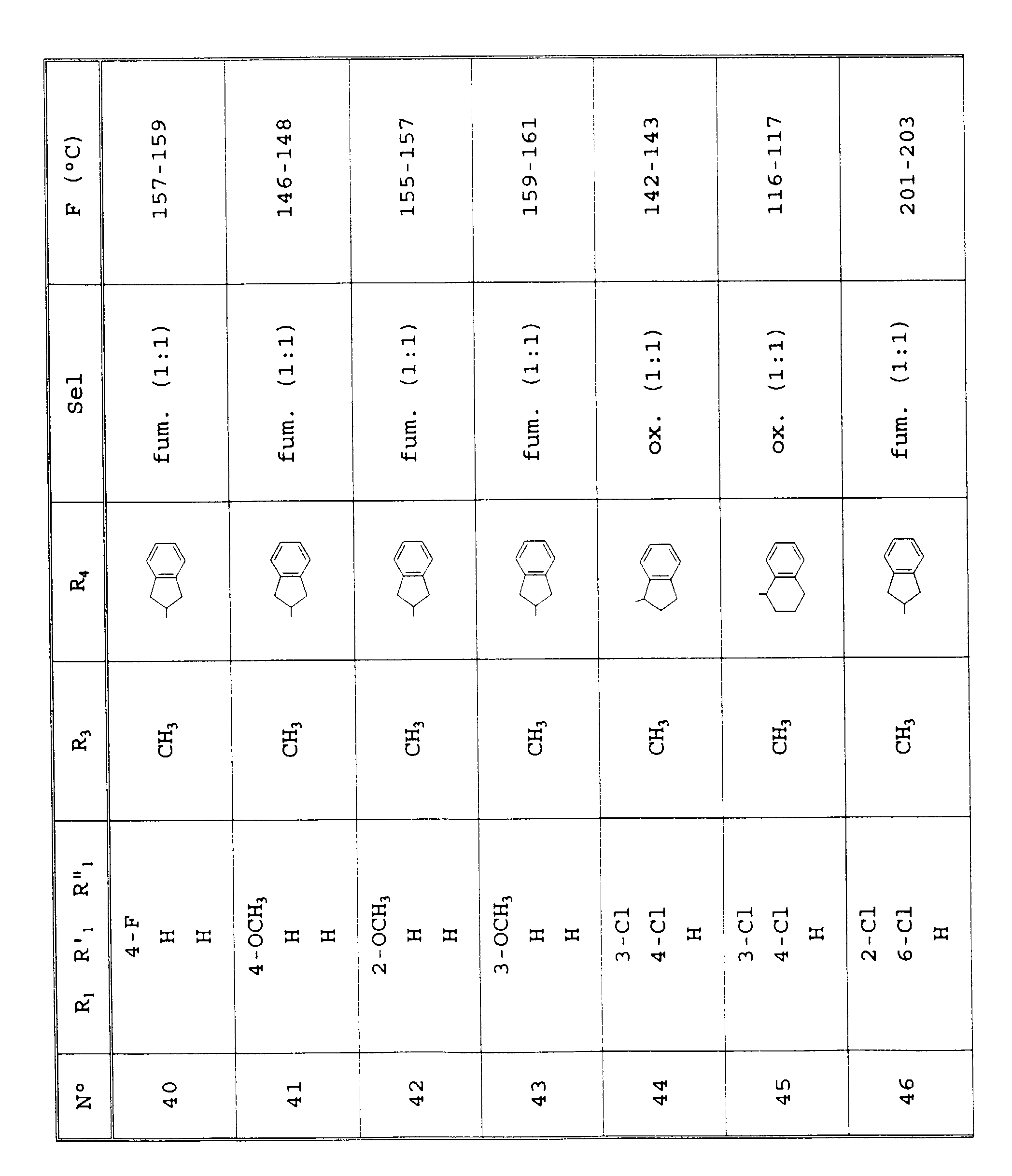

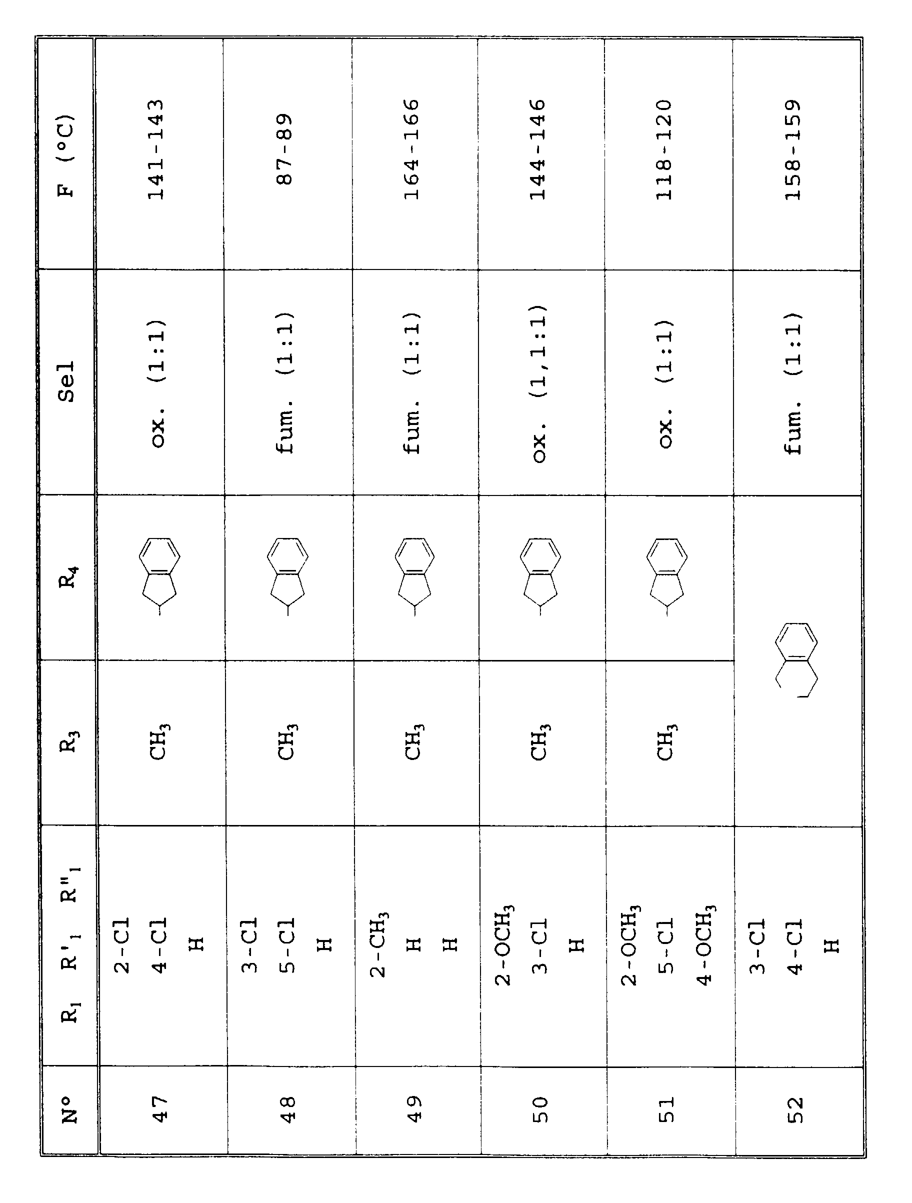

Les composés de l'invention ont fait l'objet de divers essais qui ont mis en évidence leur intérêt comme substances thérapeutiques.

Ainsi ils ont été soumis au test de l'ischémie cérébrale globale chez la souris. L'ischémie est due à un arrêt cardiaque induit par une injection intraveineuse rapide de chlorure de magnésium. Dans ce test on mesure le "temps de survie", c'est-à-dire l'intervalle entre le moment de l'injection de chlorure de magnésium et le dernier mouvement respiratoire observable de chaque souris. Ce dernier mouvement est considéré comme l'indice ultime d'une fonction du système nerveux central.
L'arrêt respiratoire apparaît approximativement 19 secondes après l'injection de chlorure de magnésium.
Des souris mâles (Swiss OF1 IFFA CREDO) sont étudiées par groupes de 10. Elles sont nourries et abreuvées ad libitum avant les essais. Le temps de survie est mesuré 10 minutes après l'administration intrapéritonéale des composés de l'invention. Les résultats sont donnés sous la forme de la différence entre le temps de survie mesuré dans un groupe de 10 souris ayant reçu le composé et le temps de survie mesuré dans un groupe de 10 souris ayant reçu le liquide véhicule.
Les rapports entre les modifications dans le terme de survie et la dose du composé sont enregistrés graphiquement selon une courbe semilogarithmique.
Cette courbe permet le calcul de la "dose efficace 3 secondes" (DE_{3''}), c'est-à-dire la dose (en mg/kg) qui produit une augmentation de 3 secondes du temps de survie par rapport au groupe témoin de 10 souris non traitées.
Une augmentation de 3 secondes du temps de survie est à la fois significative statistiquement et reproductible.
Les DE_{3''} des composés de l'invention vont de 0,1 à 30 mg/kg par voie intrapéritonéale.

Les composés de l'invention ont également fait l'objet d'une étude des courants du baryum potentiel-dépendants ("voltage-dépendent") par la technique dite du "patch-clamp".
La mesure des courants du baryum passant par les canaux calciques potentiel-dépendants est effectuée sur une préparation de cellules de cortex de rat nouveau-né (Sprague-Dawley) en culture (6 à 10 jours de culture) ; il s'agit, dans le cas de ces cellules, de courants composites mettant en jeu les canaux L, N et P, comme décrit dans *Soc. Neurosci. Abstr.* (1989) **15** 823.

Les chambres de mesure, d'un volume de 800 µl, contenant les cellules de cortex, sont placées sur la platine d'un microscope inversé Olympus IMT-2™ et les cellules sont observées au grossissement 400×. Les chambres sont continuellement perfusées (4 à 5 ml/min) à l'aide d'un dispositif distributeur de solutions acceptant 9 entrées (volume mort<50 µl) dont la sortie unique, constituée d'un tube de polyéthylène de 500 µm d'ouverture, est placée à moins de 3 mm de la cellule étudiée. Ce dispositif a l'avantage de permettre un échange rapide de solution au niveau des cellules étudiées.

La méthode de patch-clamp utilisée est décrite dans *Pfluegers Archives* (1981) **391** 85-100. Un amplificateur Axopatch-1D™ associé à un ordinateur de type AT 386-33MHz, utilisant les logiciels PCLAMP™ de Axon Instruments™ est employé pour la stimulation des cellules, l'acquisition des données et l'analyse des résultats. Pour la réalisation des enregistrements des courants du baryum, des pipettes en verre borosilicaté sont approchées des cellules grâce à un micromanipulateur hydraulique Narishige WR 60™. La pointe des pipettes est remplie de la solution intracellulaire de référence dont la composition (en mM) est la suivante : CsCl (140), CaCl₂ (1), Na₂ATP (4), EGTA (11 ; pCa=8), Hepes (10), Tris-OH (pH=7,2).
Une fois que la configuration dite "cellule" entière (whole cell) est obtenue, la cellule est perfusée avec une solution dite TEA-Baryum dont la composition (en mM) est la suivante : TEA-Cl (144), BaCl₂ (5), MgCl₂ (2), CsCl (3), glucose (10), Hepes (10), Tris-OH (pH=7,4).
Cette solution permet la mesure du courant calcique (assimilé au courant du baryum passant par les canaux calciques potentiel-dépendants) tout en s'affranchissant des courants sodiques et potassiques.
Le courant du baryum potentiel-dépendant global est obtenu par l'application d'un saut dépolarisant de potentiel d'une durée de 250 ms, portant le potentiel de membrane de -80 mV à 0 mV. La fréquence de stimulation est de 0,25 Hz.

Les composés de l'invention sont mis en solution dans le milieu TEA-baryum et appliqués une fois l'amplitude du courant du baryum stabilisée. Après obtention d'un effet inhibiteur stable, la cellule est de nouveau perfusée avec la solution TEA-baryum de contrôle afin d'observer la réversion de l'effet.

La puissance de l'effet obtenu est comparée à celle d'une solution de cadmium à 100 µM. L'inhibition du courant baryum potentiel-dépendant varie en fonction des doses de composés étudiés et, pour les composés les plus actifs, est de l'ordre de 49% à la concentration de 1 µM et de 91% à la concentration de 10 µM.

Les résultats des essais effectués sur les composés de l'invention montrent que, *in vitro*, ils ont des propriétés antagonistes calciques neuronales et, *in vivo*, ils ont des propriétés neuroprotectrices et anti-ischémiques.

Ils suggèrent que les composés peuvent être utilisés pour le traitement et la prévention de désordres cérébraux tels que ceux qui sont consécutifs, par exemple, à une attaque ischémique, un arrêt cardiaque ou respiratoire, une thrombose ou une embolie cérébrale, pour le traitement de la sénilité cérébrale, de la démence consécutive aux infarctus multiples, de la démence sénile, par exemple de la maladie d'Alzheimer ou de la maladie de Pick, pour le traitement de l'atrophie olivo-ponto-cérébellaire et d'autres maladies neurodégénératives telles que la chorée de Huntington, la sclérose latérale amyotrophique, pour le traitement des traumatismes crâniens ou spinaux, pour la prévention des dommages neuronaux faisant suite à des états convulsifs, pour le traitement de certains cancers, pour le traitement des altérations neurologiques dues au SIDA, et pour la prévention et le traitement des rétinopathies diabétiques, de la dégénérescence du nerf optique et des rétinopathies associées au glaucome, et en général pour le traitement de toute pathologie liée à un dysfonctionnement de l'homéostase calcique neuronale.

A cet effet ils peuvent être présentés sous toutes formes pharmaceutiques adaptées à l'administration entérale ou parentérale, en association avec des excipients appropriés, par exemple sous forme de comprimés, dragées, gélules, capsules, suppositoires, solutions ou suspensions buvables ou injectables, dosés pour permettre une administration journalière de 1 à 1000 mg de substance active.

## Revendications

1. Composé, éventuellement sous forme d'isomère optique pur ou de mélange de tels isomères, répondant à la formule générale (I) 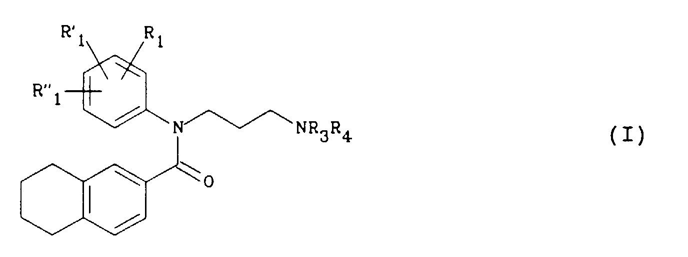 dans laquelle
R₁ représente un atome d'hydrogène ou d'halogène, un groupe méthyle ou un groupe alcoxy en C₁-C₄,
R'₁ représente un atome d'hydrogène ou d'halogène,
R"₁ représente un atome d'hydrogène ou un groupe méthoxy,
R₃, pris isolément, représente un groupe alkyle en C₁-C₃,
R₄, pris isolément, représente un groupe 2,3-dihydro-1*H*-indén-2-yle, un groupe 2,3-dihydro-1*H*-indén-1-yle ou un groupe 1,2,3,4-tétrahydronaphtalén-1-yle,
ou bien
R₃ et R₄ forment ensemble, et avec l'atome d'azote qui les porte, un groupe 1,2,3,4-tétrahydroisoquinoléin-2-yle, un groupe 6,7-diméthoxy-1,2,3,4-tétrahydroisoquinoléin-2-yle, un groupe 5,8-diméthoxy-1,2,3,4-tétrahydroisoquinoléin-2-yle, un groupe 1,2,3,4-tétrahydro-9*H*-pyrido[3,4-*b*]indol-2-yle, un groupe 1,2,3,4-tétrahydro-9*H*-pyrido[4,3-*b*]indol-3-yle, un groupe 4,5,6,7-tétrahydrothiéno[2,3-*c*]pyridin-6-yle, un groupe 2,3-dihydro-1*H*-isoindol-2-yle ou un groupe 2,3,4,5-tétrahydro-1*H*-3-benzazépin-3-yle, dont les formules respectives sont les suivantes : 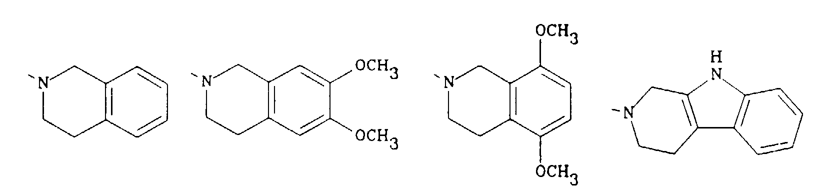 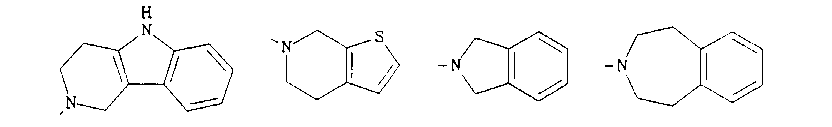 à l'état de base ou de sel d'addition à un acide,
-étant exclus les composés dans la formule desquels R₁ représente un groupe alcoxy, R'₁ et R''₁ représentent chacun un atome d'hydrogène, et R₃ et R₄ forment, avec l'atome d'azote, un groupe 1,2,3,4-tétrahydroisoquinoléin-2-yle substitué ou non substitué.

2. Composé selon la revendication 1, caractérisé en ce que R₁ représente un atome d'halogène, R'₁ représente un atome d'hydrogène ou d'halogène, R''₁ représente un atome d'hydrogène, R₃, pris isolément, représente un groupe alkyle en C₁-C₃, R₄, pris isolément, représente un groupe 2,3-dihydro-1*H*-indén-2-yle, un groupe 2,3-dihydro-1*H*-indén-1-yle ou un groupe 1,2,3,4-tétrahydronaphtalén-1-yle, ou bien R₃ et R₄ forment ensemble, et avec l'atome d'azote qui les porte, un groupe 1,2,3,4-tétrahydroisoquinoléin-2-yle, un groupe 6,7-diméthoxy-1,2,3,4-tétrahydroisoquinoléin-2-yle ou un groupe 5,8-diméthoxy-1,2,3,4-tétrahydroisoquinoléin-2-yle.

3. Le *N*-[3-[(2,3-dihydro-1*H*-indén-2-yl)méthylamino]propyl]-*N*-(3,4-dichlorophényl)-5,6,7,8-tétrahydronaphtalène-2-carboxamide, à l'état de base ou de sel d'addition à un acide.

4. Le *N*-[3-[(2,3-dihydro-1*H*-indén-1-yl)méthylamino]propyl]-*N*-(3,4-dichlorophényl)-5,6,7,8-tétrahydronaphtalène-2-carboxamide, sous forme d'isomère optique pur ou d'un mélange de tels isomères, à l'état de base ou de sel d'addition à un acide.

5. Le *N*-[3-[(1,2,3,4-tétrahydronaphtalén-1-yl)méthylamino]propyl]-*N*-(3,4-dichlorophényl)-5,6,7,8-tétrahydronaphtalène-2-carboxamide, sous forme d'isomère optique pur ou d'un mélange de tels isomères, à l'état de base ou de sel d'addition à un acide.

6. Procédé de préparation d'un composé selon la revendication 1, caractérisé en ce qu'on fait réagir une benzènamine de formule générale (II) 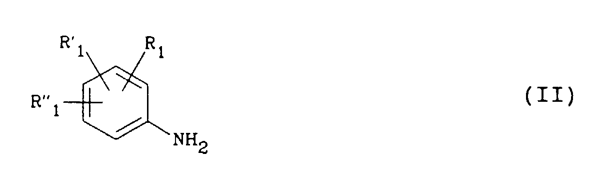 dans laquelle R₁, R'₁ et R"₁ sont tels que définis dans la revendication 1, avec le 5,6,7,8-tétrahydronaphtalène-2-carboxylate de méthyle, de formule (III) 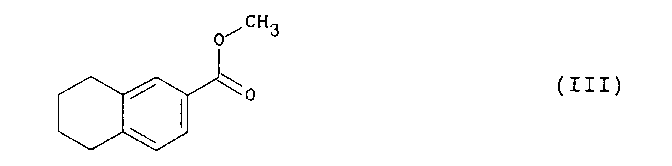 pour obtenir un amide de formule générale (IV) 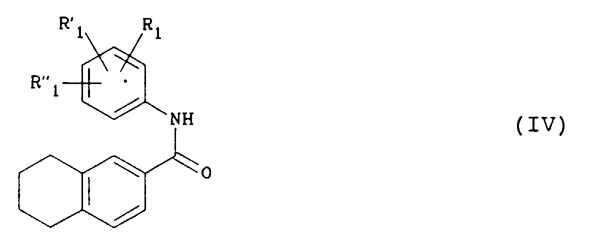 qu'on fait réagir ensuite avec le 1-bromo-3-chloropropane, de formule (V) 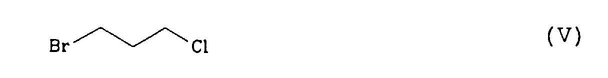 pour obtenir un dérivé chloré, de formule générale (VI) qu'on fait finalement réagir avec une amine de formule générale HNR₃R₄, dans laquelle R₃ et R₄ sont tels que définis dans la revendication 1.

7. Médicament caractérisé en ce qu'il consiste en un composé selon l'une des revendications 1 à 5.

8. Composition pharmaceutique caractérisée en ce qu'elle contient un composé selon l'une des revendications 1 à 5, associé à un excipient.

## Patentansprüche

1. Verbindung der allgemeinen Formel (I), gegebenenfalls in Form des reinen optischen Isomeren oder einer Mischung dieser Isomeren: 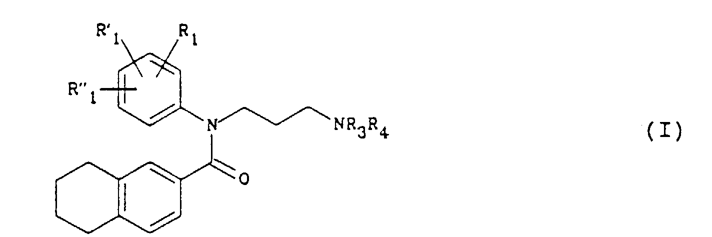 in der
R₁ ein Wasserstoffatom, ein Halogenatom, eine Methylgruppe oder eine C₁-C₄-Alkoxygruppe.
R'₁ ein Wasserstoffatom oder ein Halogenatom.
R"₁ ein Wasserstoffatom oder eine Methoxygruppe,
R₃ allein eine C₁-C₃-Alkylgruppe.
R₄ allein eine 2,3-Dihydro,1H-inden-2-yl-gruppe, eine 2,3-Dihydro-1H-inden-1-yl-gruppe oder eine 1,2,3,4-Tetrahydronaphthalin-1-yl-gruppe
oder
R₃ und R₄ gemeinsam mit dem sie tragenden Stickstoffatom eine 1,2,3,4-Tetrahydroisochinolin-2-yl,gruppe, eine 6,7-Dimethoxy-1,2,3,4-tetrahydroisochinolin-2-yl-gruppe, eine 5,8-Dimethoxy-1,2,3,4-tetrahydroisochinolin-2-yl-gruppe, eine 1,2,3,4-Tetrahydro-9H-pyrido[3,4-blindol-2-yl-gruppe, eine 1,2,3,4-Tetrahydro-9,H-pyrido[4,3-b]indol-3-yl,gruppe, eine 4,5,6,7-Tetrahydrothieno-[2,3-c]pyridin-6-yl-gruppe, eine 2,3-Dihydro-1H-isoindol-2-yl-gruppe oder eine 2,3,4,5-Tetrahydro-1*H*-3-benzazepin-3-yl-gruppe, deren Formeln die folgenden sind: 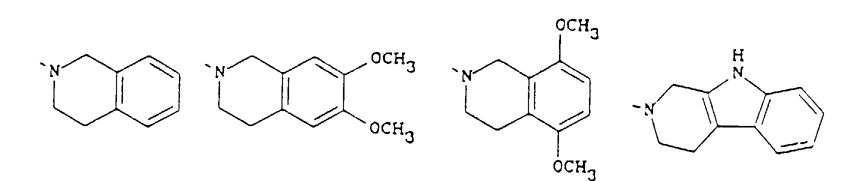 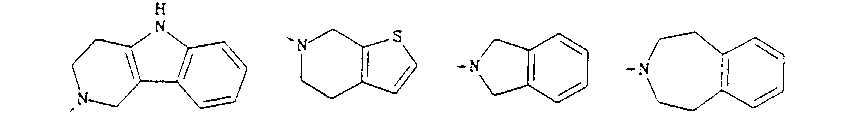 bedeuten, in Form der Base oder des Additionssalzes mit einer Säure.
- mit Ausschluß der Verbindungen der Formel, in der R₁ eine Alkoxygruppe. R'₁ und R"₁ jeweils ein Wasserstoffatom und R₃ und R₄ gemeinsam mit dem Stickstoffatom eine gegebenenfalls substituierte 1,2,3,4-Tetrahydroisochinolin-2-yl-gruppe bedeuten.

2. Verbindung nach Anspruch 1, dadurch gekennzeichnet, daß R₁ ein Halogenatom. R'₁ ein Wasserstoffatom oder ein Halogenatom, R"₁ ein Wasserstoffatom, R₃ allein eine C₁-C₃-Alkylgruppe, R₄ allein eine 2,3-Dihydro, 1H,inden-2-yl-gruppe, eine 2,3-Dihydro, 1H-inden-1-yl-gruppe oder eine 1,2,3,4-Tetrahydronaphthalin-1-yl-gruppe oder R₃ und R₄ gemeinsam mit dem sie tragenden stickstoffatom eine 1,2,3,4-Tetrahydroisochinolin-2-yl-gruppe, eine 6,7-Dimethoxy-1,2,3,4-tetrahydroisochinolin-2-yl-gruppe oder eine 5,8-Dimethoxy-1,2,3,4-tetrahydroisochinolin-2-yl-gruppe bedeuten.

3. N-[3-[(2,3-Dihydro-1H-inden-2-yl)-methylamino]-propyl)-N-(3,4-dichlorphenyl)-5,6,7,8-tetrahydronaphthalin-2-carboxamid in Form der Base oder eines Säureadditionssalzes.

4. N-[3-[(2,3,Dihydro-1H-inden-1-yl)-methylamino]-propyl]-N-(3,4-dichlorphenyl)-5,6,7,8-tetrahydronaphthalin-2-carboxamid in Form des optisch reinen Isomeren oder einer Mischung solcher Isomeren, in Form der Base oder eines Säureadditionssalzes.

5. N-[3-[(1,2,3,4-Tetrahydronaphthalin-1-yl)-methylamino]-propyll-N-(3,4-dichlorphenyl)-5,6,7,8-tetrahydronaphthalin-2-carboxamid in Form des optisch reinen Isomeren oder einer Mischung solcher Isomeren, in Form der Base oder eines Säureadditionssalzes.

6. Verfahren zur Herstellung einer Verbindung nach Anspruch 1, dadurch gekennzeichnet, daß man ein Benzolamin der allgemeinen Formel (II) 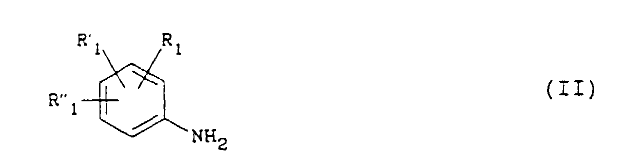 in der R₁, R'₁ und R"₁ die in Anspruch 1 angegebenen Bedeutungen besitzen, mit 5,6,7,8-Tetrahydronaphthalin-2-carbonsäuremethylester der Formel (III) 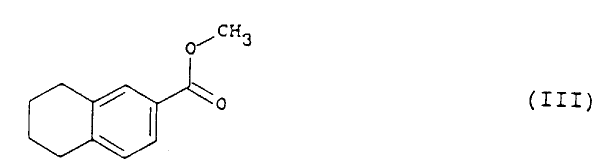 unsetzt zur Bildung eines Amids der allgemeinen Formel (IV) 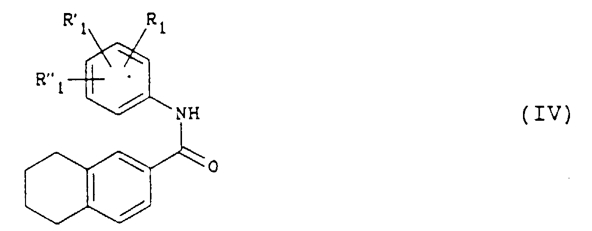 welches man anschließend mit 1-Brom-3-chlorpropan der Formel (V) 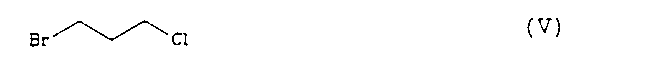 umsetzt zur Bildung eines Chlorderivats der allgemeinen Formel (VI) 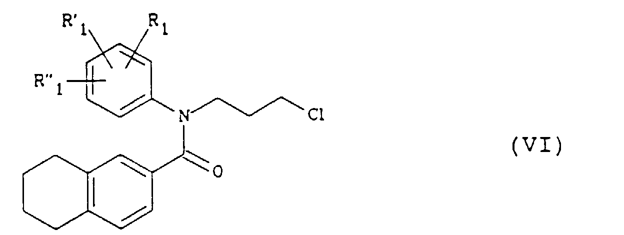 welches man mit einem Amin der allgemeinen Formel HNR₃R₄ umsetzt, in der R₃ und R₄ die in Anspruch 1 angegebenen Bedeutungen besitzen.

7. Arzneimittel, dadurch gekennzeichnet, daß es aus einer Verbindung nach einem der Ansprüche 1 bis 5 besteht.

8. Pharmazeutische Zubereitung, dadurch gekennzeichnet, daß sie eine Verbindung nach einem der Ansprüche 1 bis 5 zusammen mit einem Trägermaterial enthält.

## Claims

1. Compound, optionally in the form of a pure optical isomer or a mixture of such isomers, corresponding to the general formula (I) 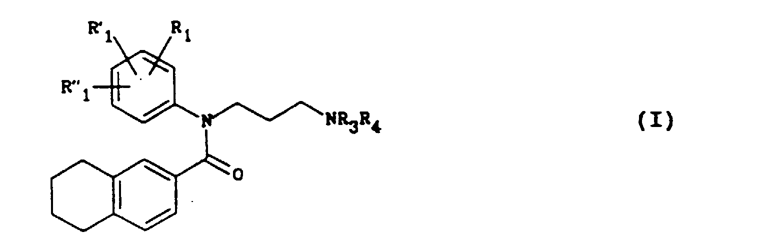 in which
R₁ represents a hydrogen or halogen atom, a -ethyl group or a C₁-C₄ alkoxy group,
R'₁ represents a hydrogen or halogen atom,
R"₁ represents a hydrogen atom or a methoxy group,
R₃, taken alone, represents a C₁-C₃ alkyl group,
R₄, taken alone, represents a 2,3-dihydro-1H-inden-2-yl group, a 2,3-dihydro-1H-inden-1-yl group or a 1,2,3,4-tetrahydronaphthalen-1-yl group,
or alternatively
R₃ and R₄ together form, with the nitrogen atom which bears them, a 1,2,3,4-tetrahydroisoquinol-2-yl group, a 6,7-dimethoxy-1,2,3,4-tetrahydroisoquinol-2-yl group, a 5,8-dimethoxy-1,2,3,4-tetrahydroisoquinol-2-yl group, a 1,2,3,4-tetrahydro-9H-pyrido[3,4-b]indol-2-yl group, a 1,2,3,4-tetrahydro-9H-pyrido[4,3-b]indol-3-yl group, a 4,5,6,7-tetrahydrothieno[2,3-c]pyrid-6-yl group, a 2,3-dihydro-1H-isoindol-2-yl group or a 2,3,4,5-tetrahydro-1H-3-benzazepin-3-yl group, the respective formulae of which are as follows: 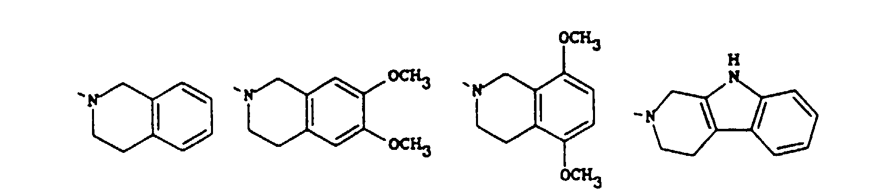 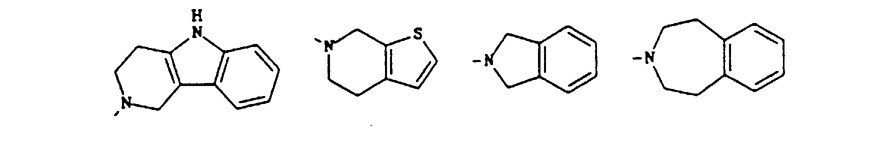 in the form of a base or an addition salt with an acid,
- those compounds being excluded in whose formula R₁ represents an alkoxy group, R'₁ and R"₁ each represent a hydrogen atom and R₃ and R₄ form, with the nitrogen atom, a substituted or unsubstituted 1,2,3,4-tetrahydroisoquinol-2-yl group.

2. Compound according to Claim 1, characterized in that R₁ represents a halogen atom, R'₁ represents a hydrogen or halogen atom, R"₁ represents a hydrogen atom, R₃, taken alone, represents a C₁-C₃ alkyl group, R₄, taken alone, represents a 2,3-dihydro-1H-inden-2-yl group, a 2,3-dihydro-1H-inden-1-yl group or a 1,2,3,4-tetrahydronaphthalen-1-yl group, or alternatively R₃ and R₄ together form, with the nitrogen atom which bears them, a 1,2,3,4-tetrahydroisoquinol-2-yl group, a 6,7-dimethoxy-1,2,3,4-tetrahydroisoquinol-2-yl group or a 5,8-dimethoxy-1,2,3,4-tetrahydroisoquinol-2-yl group.

3. N-[3-[(2,3-Dihydro-1H-inden-2-yl)methylamino]propyl]-N-(3,4-dichlorophenyl)-5,6,7,8-tetrahydronaphthalene-2-carboxamide, in the form of a base or an addition salt with an acid.

4. N-[3-[(2,3-Dihydro-1H-inden-1-yl)methylamino]propyl]-N-(3,4-dichlorophenyl)-5,6,7,8-tetrahydronaphthalene-2-carboxamide, in the form of a pure optical isomer or a mixture of such isomers, and in the form of a base or an addition salt with an acid.

5. N-[3-[(1,2,3,4-Tetrahydronaphthalen-1-yl)methylamino]propyl]-N-(3,4-dichlorophenyl)-5,6,7,8-tetrahydronaphthalene-2-carboxamide, in the form of a pure optical isomer or a mixture of such isomers, and in the form of a base or an addition salt with an acid.

6. Process for the preparation of a compound according to Claim 1, characterized in that a benzenamine of general formula (II) 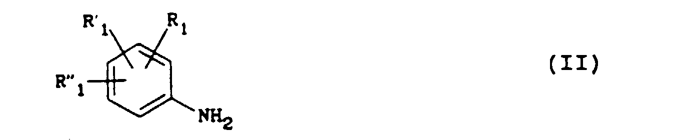 in which R₁, R'₁ and R"₁ are as defined in Claim 1, is reacted with methyl 5,6,7,8-tetrahydronaphthalene-2-carboxylate, of formula (III) 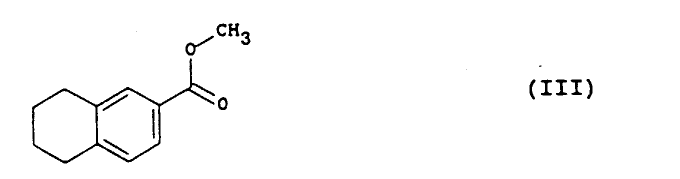 to give an amide of general formula (IV) 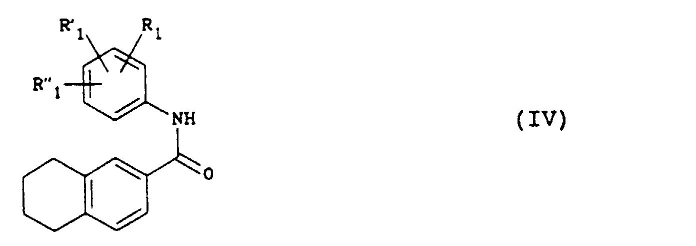 which is then reacted with 1-bromo-3-chloro-propane, of formula (V) 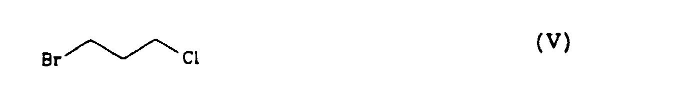 to give a chloro derivative, of general formula (VI), which is finally reacted with an amine of general formula HNR₃R₄, in which R₃ and R₄ are defined as in Claim 1.

7. Medicinal product, characterized in that it consists of a compound according to one of Claims 1 to 5.

8. Pharmaceutical composition, characterized in that it contains a compound according to one of Claims 1 to 5, combined with an excipient.
